# EUROPEAN PATENT APPLICATION

(11) **EP 4 793 352 A1**
(43) Date of publication of application: **19.08.2026**
(21) Application number: 24877127.1
(22) Date of filing: 04.10.2024
(51) Int. Cl.: C12N 15/09, A01H 5/00, A01H 5/04, C12N 15/29, C12N 15/87

(54) **GENOME EDITING METHOD AND METHOD FOR PRODUCING GENOME-EDITED PLANT**

(30) Priority: 13.10.2023 JP 2023177413
(71) Applicant: KANEKA CORPORATION, Osaka-shi, Osaka 530-8288 (JP)
(72) Inventor: MIKI, Ryuji, Iwata-shi, Shizuoka 438-0802 (JP); ICHIKAWA, Masako, Iwata-shi, Shizuoka 438-0802 (JP); HAMADA, Haruyasu, Iwata-shi, Shizuoka 438-0802 (JP)
(74) Representative: Hoffmann Eitle
(86) International application number: PCT/JP2024/035663
(87) International publication number: WO 2025/079531

(57) **Abstract**

A genome editing method includes a step of shooting fine particles coated with at least one nucleic acid and at least one protein into a shoot apex of a plant using a gene gun equipped with a stopping plate, wherein a value P represented by an equation 1, which is P = (A/2)⁵ x B × 10⁴/C³, is 0.8 or greater and 5,000 or less. In the equation 1, A is "an average particle size (µm) of the fine particles", B is "a gas pressure (psi) of the gene gun", and C is "a distance (mm) between the stopping plate of the gene gun and the shoot apex".

## Description

### TECHNICAL FIELD

The present invention relates to genome editing methods and methods for producing genome-edited plants.

### BACKGROUND ART

As a genome editing method for plants, a method using a gene gun has been known.

Patent Document 1 discloses a genome editing method for a plant, which includes a step of coating fine particles with at least one nucleic acid or at least one protein, or both at least one nucleic acid and at least one protein, a step of shooting the coated fine particles into shoot apices of embryos of ripe seeds or shoot apices of plumules of tubers using a gene gun, a step of growing the shoot apices shot with the coated fine particles to obtain plants, and a step of selecting genome-edited plants from the plants.

However, as a technology for highly efficiently performing genome editing of a plant, a sufficiently satisfactory technology has not yet been provided.

### CITATION LIST

### PATENT DOCUMENT

Patent Document 1: Japanese Unexamined Patent Application Publication No. 2017-205104

### SUMMARY OF INVENTION

### TECHNICAL PROBLEM

The present invention aims to solve the above-described various problems existing in the related art, and to achieve the following object. Specifically, an object of the present invention is to provide a method for performing genome editing or producing a genome-edited plant with high efficiency.

### SOLUTION TO PROBLEM

As a result of intensive studies to achieve the above object, the present inventors have found that a method that can perform genome editing or produce genome-edited plants with high efficiency can be provided by a genome editing method, which includes a step of shooting fine particles coated with at least one nucleic acid and at least one protein into a shoot apex of a plant using a gene gun equipped with a stopping plate, wherein a value P represented by the following equation 1 is 0.8 or greater and 5,000 or less, or a method for producing a genome-edited plant, which includes: a step of shooting fine particles coated with at least one nucleic acid and at least one protein into a shoot apex of a plant using a gene gun equipped with a stopping plate; and a step of growing the shoot apex shot with the fine particles, wherein a value P represented by the following equation 1 is 0.8 or greater and 5,000 or less. $P = {\left(A/2\right)}^{5} \times B \times {10}^{4} / {C}^{3}$

In the equation 1, A is "an average particle size (µm) of the fine particles", B is "a gas pressure (psi) of the gene gun", and C is "a distance (mm) between the stopping plate of the gene gun and the shoot apex".

The present invention is based on the insights of the present inventors, and means for solving the above problems are as follows.
<1> A genome editing method includes:
   a step of shooting fine particles coated with at least one nucleic acid and at least one protein into a shoot apex of a plant using a gene gun equipped with a stopping plate,
   wherein a value P represented by an equation 1 below is 0.8 or greater and 5,000 or less: $P = {\left(A/2\right)}^{5} \times B \times {10}^{4} / {C}^{3}$
   where, in the equation 1, A is "an average particle size (µm) of the fine particles", B is "a gas pressure (psi) of the gene gun", and C is "a distance (mm) between the stopping plate of the gene gun and the shoot apex".
<2> A method for producing a genome-edited plant, includes:
   a step of shooting fine particles coated with at least one nucleic acid and at least one protein into a shoot apex of a plant using a gene gun equipped with a stopping plate; and
   a step of growing the shoot apex shot with the fine particles,
   wherein a value P represented by an equation 1 below is 0.8 or greater and 5,000 or less: $P = {\left(A/2\right)}^{5} \times B \times {10}^{4} / {C}^{3}$
   where, in the equation 1, A is "an average particle size (µm) of the fine particles", B is "a gas pressure (psi) of the gene gun", and C is "a distance (mm) between the stopping plate of the gene gun and the shoot apex".

### EFFECTS OF INVENTION

According to the present invention, the above-described various problems existing in the related art can be solved, the object can be achieved, and there can be provided a method for performing genome editing or producing a genome-edited plant with high efficiency.

### BRIEF DESCRIPTION OF THE DRAWINGS

[Fig. 1] Fig. 1 is an example of an electrophoretic photograph analyzing the presence or absence of a target gene mutation in a lower leaf of T₀.
[Fig. 2] Fig. 2 is an example of an electrophoretic photograph analyzing the presence or absence of a target gene mutation in an upper compound leaf of T₀.
[Fig. 3] Fig. 3 is an example of an electrophoretic photograph analyzing the presence or absence of a target gene mutation in a primary leaf of T₁.
[Fig. 4] Fig. 4 is a schematic view for explaining "1. Preparation of donor plasmid used for introduction" of Example 16.
[Fig. 5] Fig. 5 is a diagram illustrating an example of the result of "5. Confirmation of introduction of gene into target site of T₁ generation plant" of Example 16.

### DESCRIPTION OF EMBODIMENTS

### (Genome editing method)

The genome editing method is a method that includes a step of shooting fine particles coated with at least one nucleic acid and at least one protein into a shoot apex of a plant using a gene gun equipped with a stopping plate, where a value P represented by the following equation 1 is 0.8 or greater and 5,000 or less. $P = {\left(A/2\right)}^{5} \times B \times {10}^{4} / {C}^{3}$

In the equation 1, A is "an average particle size (µm) of the fine particles", B is "a gas pressure (psi) of the gene gun", and C is "a distance (mm) between the stopping plate of the gene gun and the shoot apex".

The genome editing method may further include other steps A.

The genome editing method is preferably an in planta genome editing method.

The in planta genome editing method is generally a genome editing method that does not include a tissue dedifferentiation (callus formation, adventitious buds, or multiple buds) step, and is a method of performing genome editing on cells of a growth point site (definite buds and lateral buds) while a plant is in a growing state.

The genome editing is part of a technique called a new bleeding technique (NBT). The genome editing includes: disruption of a gene by cleaving a specific gene of the genome and introducing a mutation using meganuclease, CRISPR-CAS, or the like; site-specific insertion or substitution of a DNA fragment; or modification of a gene function by constructing an artificial enzyme complex in which deaminase that is a deaminating enzyme is added to a CRISPR system from which a nuclease activity has been removed, and expressing the artificial enzyme complex in a cell to highly efficiently introduce a targeted point mutation. However, the genome editing is not limited to the above, and can be any technique that can edit genomes. A target gene can be disrupted with high efficiency by using the genome editing technology. In the case of gene disruption, only the target gene can be disrupted without leaving any trace of gene recombination, and therefore such plants are not treated as a recombinant plant in some countries. In addition, according to genome editing, site-specific insertion or substitution of a DNA fragment can be efficiently performed by ligating fragments, which are homologous to sequences of both ends of a cleavage sequence, to both ends of the DNA fragment to be introduced into the site.

### <Step of shooting fine particles coated with at least one nucleic acid and at least one protein into shoot apex of plant using gene gun equipped with stopping plate>

### -At least one nucleic acid and at least one protein-

In the genome editing technology, genomic DNA is cleaved using a nuclease or a deaminase capable of targeting a cleavage site, or using guide RNA and a nuclease or a a deaminase. Thus, the genome editing technology can be distinguished from existing transformation methods, which do not use such nuclease or deaminase capable of targeting a cleavage site, or guide RNA and a nuclease or a deaminase.

In the present specification, the phrase "using a nuclease or a deaminase capable of targeting a cleavage site, or using guide RNA and a nuclease or a deaminase" means that a nuclease protein or a deaminase protein is used as one of the at least one protein, and is introduced into a cell, and means that in addition to the protein, DNA or RNA, or both DNA and RNA encoding a nuclease gene or deaminase gene as the at least one nucleic acid are used, and are introduced into a cell to express a nuclease protein or a deaminase protein. For the guide RNA, RNA may be introduced into a cell, or DNA capable of expressing guide RNA may be introduced into a cell to express guide RNA.

The nuclease or deaminase and the guide RNA may be a naturally occurring nuclease, deaminase, or guide RNA (or a combination of the foregoing), or a non-naturally occurring nuclease, deaminase, or guide RNA (or a combination of the fore going). In the present invention, expression of two or more gene products may be altered or modified. The guide RNA may include a guide sequence fused to a tracr sequence.

A lower limit value of a length (the number of bases) of the guide RNA is not particularly limited, and may be appropriately selected according to the intended purpose. The lower limit value is preferably 15 or greater, more preferably 16 or greater, yet more preferably 17 or greater, even more preferably 18 or greater, particularly preferably 19 or greater, and most preferably 20 or greater.

An upper limit value of the length (the number of bases) of the guide RNA is not particularly limited, and may be appropriately selected according to the intended purpose. The upper limit value is preferably 30 or less, more preferably 25 or less, yet more preferably 22 or less, and particularly preferably 20 or less.

The nucleic acid can include the guide RNA, the DNA capable of expressing the guide RNA, and DNA or RNA encoding the nuclease gene or the deaminase gene, or both DNA and RNA encoding the nuclease gene or the deaminase gene. Moreover, the nucleic acid may include a gene of interest to be introduced into a genome of a plant.

The nucleic acid is not particularly limited, and may be appropriately selected according to the intended purpose. Examples of the nucleic acid include RNA, DNA, a conjugate of RNA and DNA, a mixture of RNA and DNA, and the like. Among the above nucleic acids, circular RNA, linear RNA, circular DNA, linear DNA, or the like is preferable. The nucleic acid may be single stranded or double stranded. The number of the nucleic acids is not particularly limited, and may be appropriately selected according to the intended purpose.

The nucleic acid may include modification. The modification is not particularly limited, and may be appropriately selected according to the intended purpose. Examples of the modification include nuclease resistance modification, modification for improving incorporation into genomic DNA, and the like.

A length of the nucleic acid is not particularly limited, and any length of the nucleic acid can be used depending on a method to be used.

The nucleic acid may be a vector.

The vector is not particularly limited, and may be appropriately selected according to the intended purpose. Examples of the vector include pAL vectors (pAL51, pAL156, etc.), pUC vectors (pUC18, pUC19, pUC9, etc.), pBI vectors (pBI121, pBI101, pBI221, pBI2113, pBI101.2, etc.), pPZP vectors, pSMA vectors, intermediate vectors (pLGV23Neo, pNCAT, etc.), cauliflower mosaic virus (CaMV), bean golden mosaic virus (BGMV), tobacco mosaic virus (TMV), and the like.

The vector may include the gene of interest.

The gene of interest is not particularly limited, as long as expression of such a gene, or inhibition of expression of such a gene is desired. The gene of interest may be an endogenous gene or an exogenous gene of a target plant.

The exogenous gene may be a gene from a different organism species. For example, a gene from an animal, a plant, a microorganism, a virus, or the like can be used. Examples of such a gene include glucose metabolism related genes, lipid metabolism related genes, useful substance (e.g., drugs, enzymes, colorants, aromatic components, etc.) production related genes, plant growth regulation (promotion/inhibition) related genes, flowering regulation related genes, disease and pest resistance (e.g., insect resistance, nematode, mold (fungal) and bacterial disease resistance, virus (disease) resistance, etc.) related genes, environmental stress (low temperatures, high temperatures, dryness, salts, photodamages, ultraviolet rays) resistance related genes, transporter related genes, milling characteristics, bread-making characteristics, and noodle-making characteristics related genes, site-specific nuclease genes, and the like. In addition, the gene of interest may be introduced so as to express an antisense, a ribozyme, RNAi, or the like according to a purpose of the gene transfer, as well as a sense strand.

Other than the above, the vector can include, for example, a promoter, an enhancer, an insulator, an intron, a terminator, a poly(A) signal, a selectable marker gene, or the like.

A plurality of genes of interest may be used per vector. A plurality of recombinant vectors to coat the fine particles may be used per fine particle. For example, a recombinant vector including a gene of interest and a recombinant vector including a selectable marker gene may be separately produced, and mixed to coat the fine particles, and the coated fine particles may be shot into plant tissue.

The promoter may be DNA that is not derived from a plant, as long as the promoter is DNA that functions in a plant or a plant cell, and causes constitutive expression, or can induce expression in specific tissue of a plant or at a specific development stage of a plant.

Specific examples of the promoter include Cauliflower mosaic virus (CaMV) 35S promoters, El2 35S omega promoters, mopaline synthase promoters (Pnos), ubiquitin promoters derived from maize, actin promoters derived from rice, PR protein promoters derived from tobacco, ADH promoters, RuBi sco promoters, and the like.

Sequences that enhance translation activity, for example, an omega sequence of tobacco mosaic virus, can be used to increase translation efficiency. In addition, a protein may be translated from a plurality of coding regions by inserting the IRES (internal ribosomal entry site) serving as a translation initiation region into the 3'-downstream side of the promoter, and the 5'-upstream side of the translation initiation codon.

The terminator may be a sequence that can terminate the transcription of the gene transcribed by the promoter, and has a poly(A) signal. Examples of the terminator include terminators of nopaline synthase (NOS) genes, terminators of octopine synthase (OCS) genes, CaMV 35S terminators, and the like.

Examples of the selectable marker gene include herbicide resistance genes (bialaphos resistance genes, glyphosate resistance genes (EPSPS), sulfonylurea resistance genes (ALS), etc.), drug resistance genes (tetracycline resistance gene, ampicillin resistance gene, kanamycin resistance gene, hygromycin resistance gene, spectinomycin resistance gene, chloramphenicol resistance gene, neomycin resistance gene, etc.), fluorescent or luminescent reporter genes (luciferase, β-galactosidase, β-glucuronidase (GUS), green fluorescent protein (GFP), etc.), and enzyme genes, such as neomycin phosphotransferase II (NPTII), and dihydrofolate reductase. However, according to the present invention, a genome-edited plant can be produced without introducing a selectable marker gene.

The vector may be a circular plasmid, linear DNA obtained by cleaving a plasmid with a restriction enzyme or the like, a nucleic acid cassette fragment obtained by exercising only a DNA fragment to be introduced, or a DNA fragment in which 0.1 kb or greater and 1.2 kb or less of a nucleic acid is added to one or both ends of a cassette fragment. The above DNA fragments may be DNA fragments amplified by PCR. In this case, the nucleic acid to be added is not particularly limited, and may be a sequence derived from the vector, but is more preferably a sequence of a target site for introduction.

A lower limit value of a length of the nucleic acid to be added to the end of the cassette fragment is preferably 0.05 kb or greater, more preferably 0.1 kb or greater, and yet more preferably 0.2 kb. An upper limit value of the length of the nucleic acid to be added to the end of the cassette fragment is preferably 1.5 kb or less, more preferably 1.0 kb or less, and yet more preferably 0.5 kb or less.

The protein is not particularly limited, and may be appropriately selected according to the intended purpose. Examples of the protein include site-specific nucleases or deaminases, modifying enzymes, antibodies, and the like.

The site-specific nuclease is not particularly limited, and may be appropriately selected according to the intended purpose. Examples of the site-specific nuclease include: Cas proteins (Cas nucleases), such as Cas9; zinc finger nucleases; proteins that express zinc finger nuclease activity; TAL effector nucleases (TALEN); and the like.

The Cas protein can include one or more nuclear localization signals (NLS).

The Cas protein is not particularly limited, and may be appropriately selected according to the intended purpose. The Cas protein is preferably a type II CRISPR enzyme, and more preferably Cas9.

The Cas9 is not particularly limited, and may be appropriately selected according to the intended purpose. Examples of the Cas9 include Streptococcus pneumoniae (S. pneumoniae) Cas9, Streptococcus pyogenes (S. pyogenes) Cas9, S. thermophilus (S. thermophiIus) Cas9, and the like. The Cas9 may be a mutant Cas9, a Cas9 homolog, or a Cas9 ortholog.

The Cas protein may be a codon optimized for expression in a eukaryotic cell. The Cas protein may direct cleavage of one or two strands at the location of the target sequence.

A molecular weight of the protein is not particularly limited, and may be appropriately selected according to the intended purpose. The molecular weight of the protein is preferably 300 kDa or less, more preferably 200 kDa or less, and yet more preferably 150 kDa or less.

The number the nucleic acids used and the number of the proteins used are not particularly limited, and may be appropriately selected according to the intended purpose.

In addition to the nucleic acid and the protein, the fine particles may be coated with biomolecules, such as polysaccharides, lipids, and organelle, metal ions, compounds, or the like.

### -Fin particles-

The fine particles are not particularly limited, and may be appropriately selected according to the intended purpose. Examples of the fine particles include metal fine particles, ceramic fine particles, and the like. Among the above fine particles, metal fine particles are preferable.

The metal fine particles are not particularly limited, and may be appropriately selected according to the intended purpose. Examples of the metal fine particles include fine particles of a single metal, alloy particles, and the like. Among the above metal fine particles, fine particles of a single metal are preferable.

The fine particles may be aggregates of nano particles formed of a single material or a composite material.

The metal is not particularly limited, and may be appropriately selected according to the intended purpose. The metal is preferably gold, tungsten, or iron oxide, and is more preferably gold.

A lower limit value of an average particle size (diameter) of the fine particles is not particularly limited, and may be appropriately selected according to the intended purpose. The lower limit is preferably 0.3 µm or greater, more preferably 0.5 µm or greater, yet more preferably 0.6 µm or greater, even more preferably 0.8 µm or greater, and particularly preferably 1.0 µm or greater.

An upper limit value of the average particle size (diameter) of the fine particles is not particularly limited, and may be appropriately selected according to the intended purpose. The upper limit value is preferably 10 µm or less, more preferably 5.0 µm or less, yet more preferably 4.0 µm or less, even more preferably 3.0 µm or less, particularly preferably 2.0 µm or less, and most preferably 1.8 µm or less.

Among the above ranges, the average particle size (diameter) of the fine particles is preferably 0.3 µm or greater and 10 µm or less, more preferably 0.3 µm or greater and 5.0 µm or less, yet more preferably 0.3 µm or greater and 4.0 µm or less, even more preferably 0.3 µm or greater and 3.0 µm or less, particularly preferably 0.5 µm or greater and 2.0 µm or less, and most preferably 0.5 µm or greater and 1.8 µm or less.

In the present invention, the average particle size of the fine particles refers to a number average particle size. The average particle size of the fine particles can be measured by a laser diffraction and scattering method.

The average particle size of the fine particles refers to an average particle size of fine particles before being coated with at least one nucleic acid and at least one protein, i.e., an average particle size of fine particles themselves.

A density of the fine particles is not particularly limited, and may be appropriately selected according to the intended purpose. From the viewpoint of high efficiency, the density of the fine particles is preferably 5,000 kg/m³ or greater and 100,000 kg/m³ or less, more preferably 10,000 kg/m³ or greater and 50,000 kg/m³ or less, and particularly preferably 15,000 kg/m³ or greater and 25,000 kg/m³ or less.

A method of the coating is not particularly limited, and may be appropriately selected according to the intended purpose. Examples of the method include: a method in which the fine particles are washed and sterilized, and the fine particles are stirred together with at least one nucleic acid, at least one protein, CaCl₂, and spermidine using a vortex mixer, and the like.

In the case where the fine particles are coated with a protein, for example, an amphiphilic molecule and a basic compound can be used. The amphiphilic molecule (compound) includes a hydrophilic (water-soluble) portion and a hydrophobic (insoluble) portion. A hydrophilic group is a residue having affinity for water. Examples of the hydrophilic group include, but are not limited to, carbohydrates, polyoxyethylene, peptide, oligonucleotide, groups including amine, amide, alkoxy amide, carboxylic acid, or a hydroxyl group, and the like. Examples of the amphiphilic molecule (compound) include, but are not limited to, lipid molecules, liposomes, lipopolyplex, and the like. The lipopolyplex may include, for example, 1,4-bis(3-oleylaminopropyl)piperazine.

The coating may be applied on entire surfaces of the fine particles, or parts of surfaces of the fine particles.

After applying the fine particles onto a macrocarrier film as uniformly as possible using a micropipette (Pipetman) or the like, the fine particles can be dried in a sterile environment such as a clean bench.

In the case where the fine particles coated with the protein are shot, a hydrophilic macrocarrier film is preferably used as the macrocarrier film.

The hydrophilic macrocarrier film is not particularly limited, and may be appropriately selected according to the intended purpose. The hydrophilic macrocarrier film may be obtained by bonding a hydrophilic film to a macrocarrier film, or applying hydrophilic coating to a macrocarrier film. Examples of the method for hydrophilizing the film include a method using a surfactant, a photocatalyst, or a hydrophilic polymer, and the like.

The hydrophilic polymer is not particularly limited, and may be appropriately selected according to the intended purpose. Examples of the hydrophilic polymer include polyester, polyethylene glycol, hydroxyethyl methacrylate, hydroxypropyl methacrylate, dihydroxyethyl methacrylate, diethylene glycol methacrylate, triethylene glycolmethacrylate, polyethylene glycolmethacrylate, vinyl pyrrolidone, acrylic acid, acrylamide, dimethylacrylamide, glucoxyoxyethyl methacrylate, 3-sulfopropylmethacryloxyethyl dimethylammonium betaine, 2-methacryloyloxyethyl phosphorylcholine, 1-carboxydimethylmethacryloyloxyethylmethane ammonium, and the like.

A mass of the fine particle (mass per fine particle) is not particularly limited, and may be appropriately selected according to the intended purpose. The mass of the fine particle is preferably 1.26 × 10⁻¹⁵ kg or greater and 3.54 × 10⁻¹³ kg or less.

### -Gene gun-

The gene gun (also referred to as a particle gun) is not particularly limited, and may be appropriately selected according to the intended purpose, as long as the gene gun is equipped with a stopping plate.

As the gene gun, a commercially available product can be used. Examples of the commercially available product of the gene gun include Biolistic (registered trademark) PDS-1000/He Particle Delivery System (Bio-RAD) and the like.

The macrocarrier film and a plate on which a target shoot apex are placed is set in a particle gun device (gene gun) equipped with the stopping plate, and a high pressure gas is emitted from a gas acceleration tube toward the macrocarrier film. The macrocarrier film is stopped by the stopping plate, but the fine particles pass through the stopping plate and penetrate the target set below the stopping plate.

A gas pressure of the gene gun (a set value of the gas pressure set at the time of shooting) is not particularly limited, and may be appropriately selected according to the intended purpose.

A lower limit value of the gas pressure of the gene gun is not particularly limited, and may be appropriately selected according to the intended purpose. From the viewpoint of high efficiency, the lower limit value is preferably 200 psi or greater, more preferably 400 psi or greater, and particularly preferably 600 psi or greater.

An upper limit value of the gas pressure of the gene gun is not particularly limited, and may be appropriately selected according to the intended purpose. From the viewpoint of high efficiency, the upper limit value is preferably 2,000 psi or less, more preferably 1,900 psi or less, and particularly preferably 1,800 psi or less.

Among the above ranges, the gas pressure is preferably 200 psi or greater and 2,000 psi or less, more preferably 400 psi or greater and 1,900 psi or less, and most preferably 600 psi or greater and 1,800 psi or less, from the viewpoint of high efficiency.

A distance between the stopping plate of the gene gun and the shoot apex is not particularly limited, and may be appropriately selected according to the intended purpose. For example, the distance can be arbitrarily selected from a range of 20 mm to 90 mm. Within the above range, the distance is preferably 20 mm to 75 mm, more preferably 25 mm to 60 mm, yet more preferably 25 mm to 50 mm, and particularly preferably 25 mm to 45 mm, from the viewpoint of high efficiency.

In the present specification, the distance between the stopping plate of the gene gun and the shoot apex is a distance determined in the following manner.

A position of the "stopping plate of the gene gun" is a position at which a macrocarrier film is stopped at the time of shooting the fine particles into the shoot apex of the plant, and refers to a surface of the stopping filter of the stopping plate facing the shoot apex, or a line along an extension line of the surface of the stopping filter.

A position of the "shoot apex" refers to a surface of the shoot apex placed on the plate.

A perpendicular line with respect to the surface of the stopping filter facing the shoot apex or the line along the extension line of the surface is drawn from the surface of the shoot apex, and a distance from the surface of the shoot apex to the position at which the perpendicular line and the surface of the stopping filter facing the shoot apex or the line along the extension line of the surface intersect with each other is determined as the distance between the stopping plate of the gene gun and the stopping plate.

A distance between the position at which the shoot apex is placed on the plate and a center of the plate for the gene gun is not particularly limited, and may be appropriately selected according to the intended purpose. From the viewpoint of high efficiency, the distance is preferably 3 mm or greater and 15 mm or less, more preferably 6 mm or greater and 14 mm or less, and particularly preferably 8 mm or greater and 12 mm or less.

The number of times the fine particles are shot into the shoot apex by the gene gun is not particularly limited, and may be appropriately selected according to the intended purpose. Examples of the number include once, twice, three times, four times, and the like.

In the present specification, "the number of times the fine particles are shot into the shoot apex" refers to the number of times the macrocarrier film coated with the fine particles collide with the stopping plate due to the gas pressure applied (the number of times the fine particles are released from the macrocarrier film).

A lower limit value of an amount of the fine particles per shot is not particularly limited, and may be appropriately selected according to the intended purpose. From the viewpoint of high efficiency, the lower limit value is preferably 100 µg or greater, more preferably 150 µg or greater, yet more preferably 200 µg or greater, and particularly preferably 250 µg or greater.

An upper limit value of the amount of the fine particles per shot is not particularly limited, and may be appropriately selected according to the intended purpose. From the viewpoint of high efficiency, the upper limit value is preferably 1,000 µg or less, more preferably 900 µg or less, yet more preferably 800 µg or less, and particularly preferably 700 µg or less.

Within the above ranges, the amount of the fine particles per shot is preferably 100 µg or greater and 1,000 µg or less, more preferably 150 µg or greater and 900 µg or less, yet more preferably 200 µg or greater and 800 µg or less, and even more preferably 250 µg or greater and 700 µg or less.

### -Value P-

The value P is a value obtained by multiplying a 5^{th} root of 1/2 of the average particle size (µm) of the fine particles by the gas pressure (psi) of the gene gun and 10⁴, and dividing the obtained value by a 3^{rd} root of the distance (mm) between the stopping plate of the gene gun and the shoot apex. The value P is represented by the following equation 1. $P = {\left(A/2\right)}^{5} \times B \times {10}^{4} / {C}^{3}$

In the equation 1, A is "an average particle size (µm) of the fine particles", B is "a gas pressure (psi) of the gene gun", and C is "a distance (mm) between the stopping plate of the gene gun and the shoot apex".

The average particle size (µm) of the fine particles is an average particle size (µm) of the fine particles themselves.

The gas pressure (psi) of the gene gun is a set value (psi) of the gas pressure at the time of shooting the fine particles into the shoot apex of the plant.

The distance (mm) between the stopping plate of the gene gun and the shoot apex is as described above.

A lower limit value of the value P is not particularly limited, and may be appropriately selected according to the intended purpose, as long as the lower limit value is 0.8 or greater. From the viewpoint of high efficiency, the lower limit value is preferably 1 or greater, more preferably 3 or greater, yet more preferably 5 or greater, and particularly preferably 10 or greater.

An upper limit value of the value P is not particularly limited, and may be appropriately selected according to the intended purpose, as long as the upper limit value is 5,000 or less. From the viewpoint of high efficiency, the upper limit value is preferably 4,500 or less, more preferably 800 or less, yet more preferably 700 or less, even more preferably 250 or less, particularly preferably 180 or less, and most preferably 150 or less.

A range of the value P is not particularly limited, and may be appropriately selected according to the intended purpose, as long as the range of the value P is 0.8 or greater and 5,000 or less. Examples of the range include 0.8 or greater and 250 or less, greater than 250 and 5,000 or less, and the like. Moreover, the range of the value P is preferably 1 or greater and 4,500 or less, more preferably 3 or greater and 5,000 or less, yet more preferably 4 or greater and 800 or less, even more preferably 5 or greater and 700 or less, particularly preferably 10 or greater and 250 or less, particularly more preferably 10 or greater and 180 or less, and most preferably 10 or greater and 150 or less, from the viewpoint of high efficiency

### -Plant-

The plant is not particularly limited, and may be appropriately selected according to the intended purpose. Examples of the plant include angiosperms, such as monocotyledons and dicotyledons, gymnosperms, and the like.

Among the above plants, an angiosperm is preferable, and a dicotyledon is more preferable, from the viewpoint of high efficiency.

The monocotyledons are not particularly limited, and may be appropriately selected according to the intended purpose. Examples of monocotyledons include plants of the Poaceae, plants of the Liliaceae, plants of the Musaceae, plants of the Bromeliaceae, plants of the Orchidaceae, and the like.

Examples of the plants of the Poaceae include rice, wheat, barley, maize, Avena sativa, Zoysia japonica, Sorghum bicolor, ryes, millets, sugar cane, and the like. Examples of the plants of the Liliaceae include Allium fistulosum, asparagus, and the like. Examples of the plants of the Musaceae include bananas and the like. Examples of the plants of the Bromeliaceae include pineapples and the like. Examples of the plants of the Orchidaceae include orchids and the like.

The dicotyledons are not particularly limited, and may be appropriately selected according to the intended purpose. Examples of the dicotyledons include plants of the Brassicaceae, plants of the Fabaceae, plants of the Solanaceae, plants of the Cucurbitaceae, plants of the Convolvulaceae, plants of the Rosaceae, plants of the Moraceae, plants of the Malvaceae, plants of the Asteraceae, plants of the Amaranthaceae, plants of the Polygonaceae, and the like.

Among the above dicotyledons, the plants of the Fabaceae are preferable, the plants of the Glycine, the plants of the Phaseolus, the plants of the Pisum, and the plants of the Vigna are more preferable, and the plants of the Glycine are particularly preferable, from the viewpoint of high efficiency.

Examples of the plants of the Brassicaceae includes Arabidopsis thaliana, napa cabbages, Brassica rapa, cabbages, cauliflowers, Raphanus sativus var. hortensis, and the like. Examples of the plants of the Fabaceae include soybeans, adzuki beans, kidney beans, peas, cowpeas, alfalfa, and the like. Among the plants of the Fabaceae, soybeans are preferable from the viewpoint of high efficiency. Examples of the plants of the Solanaceae include tomatoes, eggplants, potatoes, tobaccos, chillis, and the like. Examples of the plants of the Cucurbitaceae include Oriental melon, cucumbers, melons, watermelons, and the like. Examples of the plants of the Convolvulaceae include Ipomoea nil, sweet potatoes (Ipomoea batatas), Calystegia pubescens, and the like. Examples of the plants of the Rosaceae include roses, strawberries, apples, and the like. Examples of the plants of the Moraceae include mulberries, figs, Hevea brasiliensis, and the like. Examples of the plants of the Malvaceae include Gossypium, Hibiscus cannabinus, and the like. Examples of the plants of the Asteraceae include lettuces and the like. Examples of the plants of the Amaranthaceae include Beta vulgaris subsp. vulgaris (sugar beet) and the like. Examples of the plants of the Polygonaceae include buckwheat and the like.

Examples of the gymnosperms include pine, Cryptomeria, Ginkgo biloba, Cycas revoluta, and the like.

The shoot apex refers to a growth point (shoot apical meristem) of a tip of a stem, and tissue of the growth point and of several leaf primordia generated from the growth point. In the present invention, only a hemispherical (dome-shaped) growth point excluding leaf primordia may be used as a shoot apex. Alternatively, a shoot apex including the growth point and leaf primordia or plant tissue including the shoot apex may be used. A virus-free tissue can be obtained by using only the growth point excluding leaf primordia.

The shoot apex is not particularly limited, and may be appropriately selected according to the intended purpose. Examples of the shoot apex include a shoot apex of an embryo of a mature seed, a shoot apex of an embryo of an immature seed, a shoot apex of a plumule of a rhizome, a shoot apex of a lateral bud of any of the foregoing, a shoot apex of an apical bud or lateral bud of a plant, a shoot apex of an apical bud or a lateral bud of a rhizome, and the like. When a treatment using a plant hormone or the like is performed to facilitate the growth of the shoot apex, such a shoot apex is included as the shoot apex, as long as a dedifferentiation step and a redifferentiation step have not occurred.

The seeds include not only natural seeds obtained by cultivating (or culturing) a plant under natural conditions or similar conditions, but also artificial seeds. However, the seeds are preferably natural seeds. If artificial seeds from which shoot apices that can be genome edited can be obtained are developed, such artificial seeds can be used. The natural seeds include not only seeds obtained in outdoor fields, but also seeds obtained by greenhouse cultivation and seeds obtained from tissue culture such as in vitro seedlings. Seeds obtained by direct reprogramming can also be used as seeds of the present invention as long as shoot apices are obtained from such seeds.

The mature seed refers to a seed that has completed the ripening process after pollination and is fully matured. The immature seed is a seed in the ripening process after pollination. The stage (the degree of maturity) of the immature seed provided to the method of the present invention is not particularly limited, and the seed may be collected at any time after pollination. The embryo is pre-germination tissue that differentiates into leaves, stems, and roots. In the present specification, the embryo also includes a plumule, a hypocotyl, and a radicle after germination.

The rhizome is a generic name for a stem that is present underground.

The rhizome is not particularly limited, and may be appropriately selected according to the intended purpose. Examples of the rhizome include a tuber, a corm, a bulb, and the like.

The tuber is a rhizome that is in a bulk form and has many buds. The corm is a rhizome that is in a spherical form and has a large apical bud. The bulb is a rhizome that is in a spherical form and has a shortened stem attached with enlarged scale-like leaves.

The plumule is a bud at an apex of an embryo of a higher plant, and can be distinguished by that it grows to become a stem above the ground after germination.

A size of the growth point (shoot apical meristem) at the tip of the stem in the shoot apex is not particularly limited, and may be appropriately selected according to the intended purpose. From the viewpoint of high efficiency, a diameter of the growth point (shoot apical meristem) at the tip of the stem is preferably 100 µm to 500 µm, more preferably 120 µm to 450 µm, and particularly preferably 140 µm to 400 µm. Examples of a plant having a diameter of the growth point (shoot apical meristem) at the tip of the stem in the above range include dicotyledons. Examples of the dicotyledons include plants of the Fabaceae, such as plants of the Glycine, plants of the Phaseolus, plants of the Pisum, and plants of the Vigna, and the like.

Examples of the size (average value) of the growth point (shoot apical meristem) of the tip of the stem are as follows. Note that, even in the same plant species, the size of the growth point (shoot apical meristem) of the tip of the stem may be slightly different between specimens.

### [Dicotyledon]

### (Fabaceae)

### <Glycine>

### -Soybean (Glycine max)-

Osuzu: 324 µm (average value of 12 measured shoot apices)
Yukihomare: 317 µm (average value of 10 measured shoot apices)
Suzumaru: 279 µm (average value of 12 measured shoot apices)
Natto small grains: 212 µm (average value of 12 measured shoot apices)

### <Phaseolus>

### -Kidney bean (Phaseolus vulgaris)-

Otebo: 287 µm (average value of 12 measured shoot apices)

### <Pisum>

### -Pea (Pisum sativum)-

Omidori: 176 µm (average value of 11 measured shoot apices)

### <Vigna>

### -Adzuki bean (Vigna angularis)-

Miyabi: 198 µm (average value of 11 measured shoot apices)

### [Monocotyledon]

### (Poaceae)

### <Zea>

### -Maize (Zea mays)-

A188: 72 µm (average value of 10 measured shoot apices)

### <Triticum>

### -Wheat (Triticum aestivum)-

Fielder: 104 µm (average value of 10 measured shoot apices)

A state of the shoot apex is not particularly limited, and may be appropriately selected according to the intended purpose. The state of the shoot apex is preferably an exposed shoot apex.

Cells of the shoot apex to be shot are not particularly limited, and may be appropriately selected according to the intended purpose. The cells are preferably L2 cells in an L2 layer, which is the second outermost layer of a shoot apical meristem.

A type of the cells is not particularly limited, and may be appropriately selected according to the intended purpose, but is preferably shoot apex stem cells to be transferred to a germline. The germline is a generic name for germ cells, which include from primordial germ cells that are a source of germ cells to final products, such as egg cells and sperm cells.

The genome editing is a process of introducing a nucleic acid, a protein, and the like into cells of the shoot apex, where the nucleic acid may, or may not be incorporated into a chromosome. According to the above process, genome-edited cells, genome-edited plants, or genome-edited progeny seeds can be obtained. The genome editing may be performed by introducing, in addition to a protein, a nucleic acid encoding a genome editing tool, such as a zinc finger nuclease, TALEN, and CRISPR-Cas, into cells of the shoot apex. The genome editing is one embodiment of transformation.

For determining genome editing efficiency, DNA is extracted from cells or plants, or both cells and plants, which have been subjected to genome editing, and whether the genome is edited in the extracted DNA is detected by PCR, electrophoresis, or Southern blotting. The genome editing efficiency is calculated from the number of shoot apices used for genome editing and the number of genome-edited plants.

For determining expression efficiency of the gene of interest, the presence or absence of the RNA or protein expressed from the gene of interest is examined. The presence or absence of the RNA can be confirmed, for example, by RT-PCR. The RNA may be detected by Northern blotting. The presence or absence of the protein can be confirmed, for example, by staining of a plant piece, electrophoresis, ELISA, RIA, dot-immunobinding assay, Western blotting, or any combination of the foregoing. The expression efficiency of the gene of interest is calculated from the number of the shoot apices used for genome editing and the number of the grown plants in which the presence of RNA or protein expressed from the gene of interest has been confirmed.

### <Other steps A>

The other steps A are not particularly limited, and may be appropriately selected according to the intended purpose. Examples of the other steps A include a step of allowing a seed to absorb water, a step of exposing a shoot apex, a step of bringing the shoot apex into contact with a hyperosmotic medium, a step of growing the shoot apex shot with the fine particles, a step of selecting a genome-edited plant from the grown plants, and the like.

### -Step of allowing seed to absorb water-

The water absorption is not particularly limited, and may be appropriately selected according to the intended purpose. Examples of the water absorption include a method in which seeds are soaked in water and the soaked seeds are incubated, and the like.

A temperature of the incubation is not particularly limited, and may be appropriately selected according to the intended purpose. The temperature is preferably 10°C or higher and 40°C or lower, more preferably 15°C or higher and 35°C or lower, yet more preferably 15°C or higher and 30°C or lower, and particularly preferably 15°C or higher and 25°C or lower.

A duration of the water absorption is not particularly limited, and may be appropriately selected according to the intended purpose or conditions of seeds. The duration is preferably 3 hours or longer, more preferably 6 hours or longer, yet more preferably 12 hours or longer, particularly preferably 14 hours or longer, and most preferably 16 hours or longer.

An upper limit value of the duration of the water absorption is not particularly limited, and may be appropriately selected according to the intended purpose or conditions of seeds. The upper limit value is preferably 240 hours or shorter, more preferably 120 hours or shorter, yet more preferably 48 hours or shorter, particularly preferably 36 hours or shorter, and most preferably 24 hours shorter.

The seeds are softened by the water absorption so that shoot apices can be easily exposed.

In the step of allowing the seed to absorb water, the water may be replaced one or more times.

### -Step of exposing shoot apex-

A method of exposing shoot apices of embryos of the mature seeds and the immature seeds is not particularly limited, and may be appropriately selected according to the intended purpose. Examples of the method include a method of removing coleoptiles and leaf primordia, a method of removing seed coats and cotyledons, and the like.

A method of exposing a shoot apex of a plumule of the rhizome is not particularly limited, and may be appropriately selected according to the intended purpose. Examples of the method include a method of removing leaf primordia.

A method of exposing a shoot apex of the apical bud or lateral bud is not particularly limited, and may be appropriately selected according to the intended purpose. Examples of the method include a method of removing leaf primordia.

Specifically, in the case of wheat, barley, rice, or maize, a shoot apex can be exposed by removing a coleoptile and leaf primordia. In the case of soybean, a shoot apex can be exposed by removing a seed coat and cotyledons. In the case of a potato, a shoot apex can be exposed by allowing a seed potato to sprout, cutting out a plumule, and removing leaf primordia from the plumule. In the case of an apple, a shoot apex can be exposed by removing leaf primordia from a seed embryo or a harvested apical bud or lateral bud.

The exposing device is not particularly limited, and may be appropriately selected according to the intended purpose, as long as the coleoptiles, leaf primordia, seed coats, cotyledons, and any combination of the foregoing can be removed under a stereoscopic microscope. Examples of the exposing device include: piercing tools, such as a needle having a diameter of approximately 0.2 mm; tweezers; pipettes; syringes; and cutting tools, such as scalpels, cutters, and the like.

In order to obtain a virus-free shoot apex, the scalpel may be replaced with a fresh sterile scalpel at the final stage of cutting out the shoot apex. In this case, a virus-free genome-edited plant can be obtained.

After exposing the shoot apex, the endosperm and the excess scutellum portion are cut by a cutting tool, such as a scalpel, and the embryo and scutellum including the exposed shoot apex can be placed onto an agar medium with the shoot apex facing upward.

### -Step of bringing shoot apex into contact with hyperosmotic medium-

The step of bringing the shoot apex into contact with a hyperosmotic medium is a step of performing a hyperosmotic treatment on the shoot apex.

A method of bringing the shoot apex into contact with the hyperosmotic medium is not particularly limited, and may be appropriately selected according to the intended purpose. Examples of the method include a method of immersing the shoot apex of the plant in the hyperosmotic medium, a method of placing the shoot apex of the plant onto the hyperosmotic medium, a method of spraying the hyperosmotic medium onto the shoot apex of the plant.

An environment in which the hyperosmotic treatment is performed may be a bright place or a dark place. From the viewpoint of germination efficiency, the hyperosmotic treatment is preferably performed in the dark in case of a dark germinator, and is preferably performed under light in case of a light germinator.

Examples of the hyperosmotic medium include a medium including a sugar or a sugar alcohol, and the like.

Examples of the sugar or the sugar alcohol include sorbitol, mannitol, sucrose, and the like. From the viewpoint of efficient hyperosmotic treatment, the medium preferably includes sorbitol, mannitol, and sucrose.

Examples of an osmotic potential of the hyperosmotic medium include a potential that is equal to at least an osmotic potential of an 11% sucrose aqueous solution, and the like.

To the hyperosmotic medium, various gelling agents can be added. The gelling agent is not particularly limited, and may be appropriately selected according to the intended purpose. Examples of the gelling agent include agarose, agar, gellan gum, gelrite, phytagel, and the like. A concentration of the gelling agent in the hyperosmotic medium is not particularly limited, and may be appropriately selected according to a type of the gelling agent. In the case of phytagel, for example, the concentration is preferably 1.0 g/L to 10.0 g/L, and more preferably 2.0 g/L to 8.0 g/L.

The osmotic pressure of the hyperosmotic medium before adding the gelling agent is not particularly limited, and may be appropriately selected according to the intended purpose. The osmotic pressure is preferably 400 mOsmol/kg or greater and 2,000 mOsmol/kg or less, and more preferably 400 mOsmol/kg or greater and 1,500 mOsmol/kg or less.

A timing of the hyperosmotic treatment is not particularly limited, and may be appropriately selected according to the intended purpose. Examples of the timing include before or after, or both before and after the step of shooting the fine particles coated with the nucleic acid and the protein into the shoot apex of the plant using the gene gun equipped with the stopping plate.

Among the above timings, the hyperosmotic treatment is preferably performed before and after the step of shooting the fine particles coated with the nucleic acid and the protein into the shoot apex of the plant using the gene gun equipped with the stopping plate, from the viewpoint of high efficiency.

A duration of the hyperosmotic treatment is not particularly limited, and may be appropriately selected according to the intended purpose. When the hyperosmotic treatment is performed before the step of shooting the fine particles coated with the nucleic acid and the protein into the shoot apex of the plant using the gene gun equipped with the stopping plate from the viewpoint of high efficiency, the duration of the hyperosmotic treatment is preferably 1 hour or longer and 60 hours or shorter, more preferably 1 hour or longer and 48 hours or shorter, and yet more preferably 2 hours or longer and 48 hours or shorter. When the hyperosmotic treatment is performed after the step of shooting the fine particles coated with the nucleic acid and the protein into the shoot apex of the plant using the gene gun equipped with the stopping plate from the viewpoint of high efficiency, the duration of the hyperosmotic treatment is preferably 1 hour or longer and 60 hours or shorter, more preferably 1 hour or longer and 48 hours or shorter, and yet more preferably 2 hours or longer and 48 hours or shorter. When the duration is within the preferable range, it is advantageous in that transformation or the like can be performed with high efficiency.

### -Step of growing shoot apex shot with fine particles-

A method of the growing is not particularly limited, and may be appropriately selected according to the intended purpose. Examples of the method include a method in which the shoot apex shot with the fine particles is grown on an agar medium for approximately one month, followed by transplanting the growing plant to a soil, and the like.

### -Step of selecting genome-edited plant from grown plants-

In the genome editing method, a genome-edited plant can be obtained by growth in a normal medium without applying selection pressure by a drug or the like (without antibiotics or the like), but can be also obtained by selectively culturing genome-edited cells using a drug.

In the case where selection pressure is applied by a drug or the like, a drug-resistance gene can be introduced in addition to the gene of interest.

The drug is not particularly limited, and may be appropriately selected according to the intended purpose. Examples of the drug include chlorsulfuron (resistance can be obtained by introduction of a mutant ALS gene (acetolactate synthase gene)), which is a sulfonyl urea herbicide, and the like.

### (Method for producing genome-edited plant)

The method for producing a genome-edited plant includes: a step of shooting fine particles coated with at least one nucleic acid and at least one protein into a shoot apex of a plant using a gene gun equipped with a stopping plate; and a step of growing the shoot apex shot with the fine particles, where a value P represented by the following equation 1 is 0.8 or greater and 5,000 or less. $P = {\left(A/2\right)}^{5} \times B \times {10}^{4} / {C}^{3}$

In the equation 1, A is "an average particle size (µm) of the fine particles", B is "a gas pressure (psi) of the gene gun", and C is "a distance (mm) between the stopping plate of the gene gun and the shoot apex".

The method for producing the genome-edited plant may further include other steps B.

The step of shooting the fine particles coated with at least one nucleic acid and at least one protein into the shoot apex of a plant using the gene gun equipped with the stopping plate is as described in (Genome editing method) above.

The step of growing the shoot apex shot with the fine particles is as described in <Other steps A> of (Genome editing method) above.

### <Other steps B>

The other steps B are not particularly limited, and may be appropriately selected according to the intended purpose. Examples of the other steps B include a step of allowing a seed to absorb water, a step of exposing a shoot apex, a step of bringing the shoot apex into contact with a hyperosmotic medium, a step of selecting a genome-edited plant from the grown plants, and the like.

The step of allowing the seed to absorb water, the step of exposing the shoot apex, the step of bringing the shoot apex into contact with the hyperosmotic medium, and the step of selecting the genome-edited plant from the grown plants are as described in <Other steps A> of (Genome editing method) above.

### [Examples]

Examples of the present invention will be described hereinafter, but the present invention is not limited by these examples in any way.

### (Comparative Example 1) Targeted mutagenesis 1 by introduction of guide RNA (gRNA) and Cas9

In order to produce genome-edited specimens, a complex of gRNA and Cas9 was introduced into shoot apices using a particle gun. Then, a yield of the genome-edited specimens was determined. For the determination of the yield of the genome-edited specimens, the complex of gRNA and Cas9, which targeted the gene Glyma.08g116300, was introduced into shoot apices of soybean.

### 1. Confirmation of introduction of target gene mutation in T₀ generation plant (1) Preparation of mature seeds of soybean

Mature seeds of soybean (Yukihomare) were immersed in Kitchen Haiter (hypochlorous acid concentration: 6%, Kao Corporation), and were agitated for 2 minutes at room temperature to sterilize the mature seeds, followed by washing the mature seeds with tap water. The sterilized mature seeds were transferred into a sealed container, and were soaked in sterile water to absorb water. The resultant seeds were incubated in an incubator of 20°C for approximately 24 hours, and then were provided to the following experiment.

### (2) Exposure of shoot apex of mature seed embryo

Under a stereoscopic microscope, the seed coat and the cotyledons were all removed by a sterilized knife (a sterilized scalpel) to leave only a hypocotyl. Then, a primary leaf and a base of the hypocotyl were removed using a tip of a needle (diameter: 0.20 mm) to fully expose a shoot apex portion. The shoot apex portions exposed in the above manner were placed on a BM medium (4.3 g/L MS salt, MS vitamin, 3% sucrose, 0.50 g/L MES, 3% PPM (plant preservative mixture, NACALAI TESQUE, INC.), 6.0 g/L Phytagel (registered trademark, Sigma-Aldrich Co., LLC.), pH 5.7) so that approximately 20 shoot apex portions were provided per plate.

### (3) Introduction of gRNA and Cas9

Introduction of gRNA and Cas9 into the shoot apices of the mature embryos was performed by particle bombardment in the following manner.

As fine particles, 30 mg of gold particles having an average particle size of 0.6 µm were weighed and added to 500 µL of 70% ethanol, and the resultant mixture was sufficiently suspended by vortexing. Thereafter, the gold particles were precipitated by centrifugation, and the ethanol was removed. Thereafter, 250 µL of sterile water was added to prepare a sterilized gold particle solution.

As the gRNA, a mixture (1.0 µg/µL) of crRNA and tracrRNA (Fasmac Co., Ltd.) was used. As the Cas9, Cas9 derived from Streptococcus pyogenes (Takara Bio Inc.) was used.
crRNA: ACCCAAGUAAAGUACCAAGGguuuuagagcuaugcuguuuug (SEQ ID NO: 1)
tracrRNA:
   aaacagcauagcaaguuaaaauaaggcuaguccguuaucaacuugaaaaaguggcaccgagucggugcu (SEQ ID NO: 2)

Five microliters of the gRNA mixture, 13 µg of the Cas9, 4 µL of 10×Cut Smart (registered trademark) Buffer (New England Biolabs), and 1.0 µL of Ribolock RNase Inhibitor (ThermoFisher) were added, and the resultant mixture was filled up to 20 µL in total with sterile distilled water. After leaving the resultant mixture to stand at room temperature for 10 minutes, 5 µL of 1,4-bis(3-oleoylamidopropyl)piperazine/histone H1 protein mixture (3:1, 1,330 µg/mL) was added, and the resultant mixture was left to stand at room temperature for 5 minutes. To the mixture, 1,800 µg of the gold particles were added, and the resultant mixture was left to stand on ice for 10 minutes. After discarding the supernatant, 24 µL of Nuclease-Free Water was added, and the resultant was provided as a gold particle/Cas9 composite. An amount of the gold particles per shot was set to 450 µg. In a clean bench, a hydrophilic film (3M, SH2CLHF) cut into a square having a side of 1.5 cm was adhered to a center of a macrocarrier. To each hydrophilic film, 6 µL of the gold particle/Cas9 composite was poured, and was air-dried.

As the particle gun, Biolistic (registered trademark) PDS-1000/He Particle Delivery System (Bio-RAD) was used.

The pressure (gas pressure) at the time of shooting was set to 1,350 psi (approximately 94.9 kgf/cm²), and the distance from the stopping plate to the target tissue (shoot apices) was set to 50 mm. With the above conditions, the value P represented by the equation 1 was 0.26.

The fine particles were shot once per dish, and then the resultant tissue was allowed to stand overnight in the dark.

### 2. Investigation using genomic PCR of T₀ generation plant as indicator (1) Growth of RNA and Cas9-introduced specimens

The gRNA and Cas9-introduced specimens, which had been left to stand overnight, were transferred into a disposable plant cell culture container in which an RM medium (4.3 g/L MS salt, MS vitamin, 3% sucrose, 0.50 g/L MES, 3% PPM (plant preservative mixture, NACALAI TESQUE, INC.), 3.0 g/L gelrite, pH 5.7) was added, and were allowed to grow in an incubator (25°C, a day length of 16 hours). When the growth of roots and differentiation of leaves from the shoot apices were observed, the specimens were transplanted to a cell tray containing a gardening seedling soil. Thereafter, the plants were grown in the same incubator until the third true leaves appeared.

### (2) Confirmation of introduction of target gene mutation in T₀ lower leaf

Whether the mutation was introduced into the target gene in the obtained plant was determined by the CAPS assay. Three independent leaves were selected and collected from the leaves up to the third true leaves, and genomic DNA was extracted from the collected leaves by a benzyl chloride method. By using the extracted genomic DNA as a template, PCR was performed with primers specific to the gene Glyma.08g116300.
Primer sequence: ATGTGTCGCAGCAAATCAAAATGG (SEQ ID NO: 3)
Primer sequence: ACGCCCAACCGCTTCCTATA (SEQ ID NO: 4)

The PCR reaction solution was prepared by adding 20 ng of the above genomic DNA, 0.2U of KOD FX Neo (TOYOBO CO., LTD.), 5 µL of 2× the accompanying buffer, 2 µL of 2 mM dNTPs, and 0.2 µM each of the primer pair, and filling the resultant mixture up to 10 µL in total with sterile distilled water.

The PCR was performed using ProFlex PCR System (trademark, ThermoFisher) at 94°C for 2 minutes, and at 98°C for 10 seconds, followed by at 68°C for 30 seconds. The above reaction was determined as one cycle, and 35 cycles of the reaction were performed. After the PCR, 1.7 µL of each PCR product, 0.5 µL of 10× the accompanying buffer, and 5U of an appropriate restriction enzyme were added together, and the resultant mixture was filled up to 5 µL with sterile distilled water. After reacting the mixture at an optimum temperature for 3 hours, 2.0% agarose electrophoresis was performed, followed by staining with GelRed.

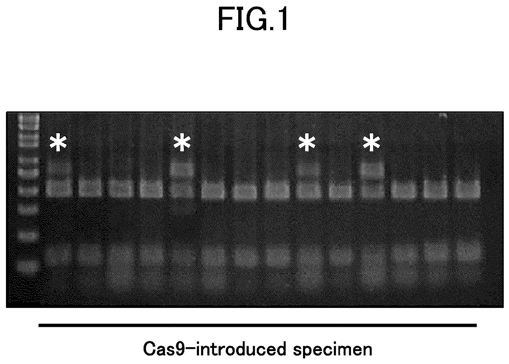

In a wild-type strain, the PCR product (505 bp) was completely cleaved (120 bp and 385 b) by the restriction enzyme, whereas in the specimen in which the mutation had been introduced, the PCR product included a residue without being cleaved (see Fig. 1; the PCR product of the specimen in which the mutation was introduced into the target gene by genome editing included a residue without being cleaved, as the samples indicated by asterisks in Fig. 1).

In the present example, among 40 shoot apex specimens, the specimen in which the mutation was introduced into the target gene was 0, and a genome-edited specimen could not be confirmed under the above conditions.

### (Example 1) Targeted mutagenesis 2 by introduction of gRNA and Cas9

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Comparative Example 1, expect that the hypocotyls with the fully-exposed shoot apex portions were placed on the BM medium at approximately 16 per plate.

### (3) Introduction of gRNA and Cas9

The gRNA and Cas9 were introduced in the same manner as in (3) of 1 of Comparative Example 1, except that the pressure (gas pressure) at the time of shooting was set to 1,550 psi (approximately 108.9 kgf/cm²), and the distance between the stopping plate and the target tissue (shoot apex) was set to 35 mm. With the above conditions, the value P represented by the equation 1 was 0.88.

### 2. Investigation using genomic PCR of T₀ generation plant as indicator (1) Growth of RNA and Cas9-introduced specimens

The RNA and Cas9-introduced specimens were grown in the same manner as in (1) of 2 of Comparative Example 1.

### (2) Confirmation of introduction of target gene mutation in T₀ lower leaf

Whether the mutation was introduced into the target gene in the obtained plant was determined according to the method described in (2) of 2 of Comparative Example 1. As a result, among 192 shoot apex specimens, genome editing was confirmed in 18 specimens (mutation introduction efficiency: 9.3%) in the present example (see Fig. 1).

### (3) Confirmation of introduction of target gene mutation in T₀ upper compound leaf

The above 18 specimens in which the target gene mutation was confirmed were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

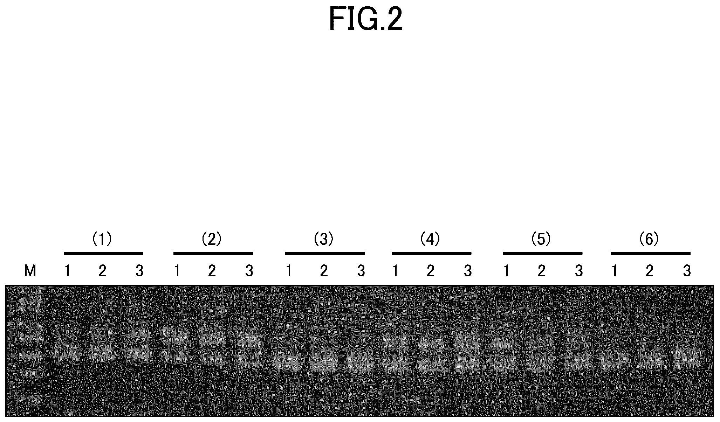

As a result, the target gene mutation was detected in several upper compound leaves of 10 grown specimens (see Fig. 2). The mutation introduction efficiency for the 192 shoot apex specimens was 5.2%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant

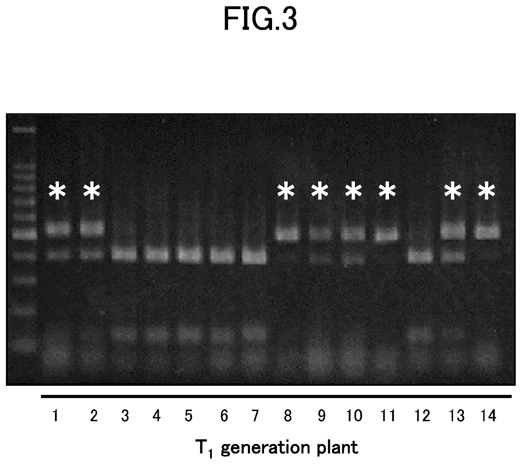

T₁ seeds harvested from the T₀ plant were sown, primary leaves were sampled, and genomic DNA was extracted from the primary leaves by the benzyl chloride method. Then, the confirmation of introduction of the target gene mutation was performed and evaluated by the CAPS assay according to the method described in (2) of 2 of Comparative Example 1. As a result, the introduction of the target gene mutation was confirmed in the T₁ generation primary leaf of one line of the T₁ generation (mutation introduction efficiency: 0.52%) (see Fig. 3).

In Fig. 3, the samples indicated with asterisks are the T₁ plants in which the target gene mutation is introduced by genome editing.

### (Example 2) Targeted mutagenesis 3 by introduction of gRNA and Cas9

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 1.

### (3) Introduction of gRNA and Cas9

The gRNA and Cas9 were introduced in the same manner as in (3) of 1 of Example 1, except that the pressure (gas pressure) at the time of shooting was set to 1,800 psi (approximately 126.5 kgf/cm²). With the above conditions, the value P represented by the equation was 1.0.

### 2. Investigation using genomic PCR of T₀ generation plant as indicator (1) Growth of RNA and Cas9-introduced specimens

The RNA and Cas9-introduced specimens were grown in the same manner as in (1) of 2 of Comparative Example 1.

### (2) Confirmation of introduction of target gene mutation in T₀ lower leaf

Whether the mutation was introduced into the target gene in the obtained plant was determined according to the method described in (2) of 2 of Comparative Example 1. As a result, among 176 shoot apex specimens, genome editing was confirmed in 21 specimens (mutation introduction efficiency: 11.9%) in the present example (see Fig. 1).

### (3) Confirmation of introduction of target gene mutation in T₀ upper compound leaf

The above 21 specimens in which the target gene mutation was confirmed were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 12 grown specimens (see Fig. 2). The mutation introduction efficiency for the 176 shoot apex specimens was 6.8%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant

T₁ seeds harvested from the T₀ plant were sown, primary leaves were sampled, and genomic DNA was extracted from the primary leaves by the benzyl chloride method. Then, the confirmation of introduction of the target gene mutation was performed and evaluated by the CAPS assay according to the method described in (2) of 2 of Comparative Example 1. As a result, the introduction of the target gene mutation was confirmed in the T₁ generation primary leaf of one line of the T₁ generation (mutation introduction efficiency: 0.57%) (see Fig. 3).

### (Example 3) Targeted mutagenesis 4 by introduction of gRNA and Cas9

### (hyperosmotic treatment 1)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Comparative Example 1, except that, before shooting of the fine particles, fully exposed shoot apex portions were placed onto a permeable bombardment medium (SOY-OSMO: 4.3 g/L MS salt, MS vitamin, 2% sucrose, 3.64% mannitol, 3.64% sorbitol, 0.70 g/L L-proline, 3% PPM (plant preservative mixture, NACALAI TESQUE, INC.), 2.0 mL/L meropenem trihydrate, 4.0 g/L phytagel, pH 5.8) at approximately 16 per plate, and were incubated in the dark at 20°C for 4 hours.

### (3) Introduction of gRNA and Cas9

The gRNA and Cas9 were introduced in the same manner as in (3) of 1 of Example 1, except that the pressure (gas pressure) at the time of shooting was set to 1,800 psi (approximately 126.5 kgf/cm²). With the above conditions, the value P represented by the equation was 1.0.

### 2. Investigation using genomic PCR of T₀ generation plant as indicator

### (1) Growth of RNA and Cas9-introduced specimens

The RNA and Cas9-introduced specimens were grown in the same manner as in (1) of 2 of Comparative Example 1.

### (2) Confirmation of introduction of target gene mutation in T₀ lower leaf

Whether the mutation was introduced into the target gene in the obtained plant was determined according to the method described in (2) of 2 of Comparative Example 1. As a result, among 128 shoot apex specimens, genome editing was confirmed in 23 specimens (mutation introduction efficiency: 17.9%) in the present example (see Fig. 1).

### (3) Confirmation of introduction of target gene mutation in T₀ upper compound leaf

The twenty-three specimens in which the target gene mutation was confirmed were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 16 grown specimens (see Fig. 2). The mutation introduction efficiency for the 128 shoot apex specimens was 12.5%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant

T₁ seeds harvested from the T₀ plant were sown, primary leaves were sampled, and genomic DNA was extracted from the primary leaves by the benzyl chloride method. Then, the confirmation of introduction of the target gene mutation was performed and evaluated by the CAPS assay according to the method described in (2) of 2 of Comparative Example 1. As a result, the introduction of the target gene mutation was confirmed in the T₁ generation primary leaf of 3 lines of the T₁ generation (mutation introduction efficiency: 2.3%) (see Fig. 3).

### (Example 4) Targeted mutagenesis 5 by introduction of gRNA and Cas9 (hyperosmotic treatment 2)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.0 µm were used, and the pressure (gas pressure) at the time of shooting was set to 1,800 psi (approximately 126.5 kgf/cm²). With the above conditions, the value P represented by the equation 1 was 13.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ lower leaf

Whether the mutation was introduced into the target gene in the obtained plant was determined according to the method described in (2) of 2 of Comparative Example 1. As a result, among 96 shoot apex specimens, genome editing was confirmed in 47 specimens (mutation introduction efficiency: 49%) in the present example (see Fig. 1).

### (3) Confirmation of introduction of target gene mutation in T₀ upper compound leaf

The 47 specimens in which the target gene mutation was confirmed were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 29 grown specimens (see Fig. 2). The mutation introduction efficiency for the 96 shoot apex specimens was 30.2%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant

T₁ seeds harvested from the T₀ plant were sown, primary leaves were sampled, and genomic DNA was extracted from the primary leaves by the benzyl chloride method. Then, the confirmation of introduction of the target gene mutation was performed and evaluated by the CAPS assay according to the method described in (2) of 2 of Comparative Example 1. As a result, the introduction of the target gene mutation was confirmed in the T₁ generation primary leaf of 14 lines of the T₁ generation (mutation introduction efficiency: 14.6%) (see Fig. 3).

### (Example 5) Targeted mutagenesis 6 by introduction of gRNA and Cas9 (hyperosmotic treatment 3)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, and the pressure (gas pressure) at the time of shooting was set to 900 psi (approximately 63.2 kgf/cm²). With the above conditions, the value P represented by the equation 1 was 69.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ lower leaf

Whether the mutation was introduced into the target gene in the obtained plant was determined according to the method described in (2) of 2 of Comparative Example 1. As a result, among 32 shoot apex specimens, genome editing was confirmed in 9 specimens (mutation introduction efficiency: 28.1%) in the present example (see Fig. 1).

### (3) Confirmation of introduction of target gene mutation in T₀ upper compound leaf

The above 9 specimens in which the target gene mutation was confirmed were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 3 grown specimens (see Fig. 2). The mutation introduction efficiency for the 32 shoot apex specimens was 9.3%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant

T₁ seeds harvested from the T₀ plant were sown, primary leaves were sampled, and genomic DNA was extracted from the primary leaves by the benzyl chloride method. Then, the confirmation of introduction of the target gene mutation was performed and evaluated by the CAPS assay according to the method described in (2) of 2 of Comparative Example 1. As a result, the introduction of the target gene mutation was confirmed in the T₁ generation primary leaf of one line of the T₁ generation (mutation introduction efficiency: 3.1%) (see Fig. 3).

### (Example 6) Targeted mutagenesis 7 by introduction of gRNA and Cas9 (hyperosmotic treatment 4)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, the pressure (gas pressure) at the time of shooting was set to 1,800 psi (approximately 126.5 kgf/cm²), and the distance from the stopping plate to the target tissue (shoot apices) was set to 50 mm. With the above conditions, the value P represented by the equation 1 was 47.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 12 grown specimens (see Fig. 2). The mutation introduction efficiency for the 64 shoot apex specimens was 18.8%.

### (Example 7) Targeted mutagenesis 8 by introduction of gRNA and Cas9 (hyperosmotic treatment 5)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, and the pressure (gas pressure) at the time of shooting was set to 650 psi (approximately 45.7 kgf/cm²). With the above conditions, the value P represented by the equation 1 was 49.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 15 grown specimens (see Fig. 2). The mutation introduction efficiency for the 96 shoot apex specimens was 15.6%.

### (Example 8) Targeted mutagenesis 9 by introduction of gRNA and Cas9 (hyperosmotic treatment 6)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, and the pressure (gas pressure) at the time of shooting was set to 1,100 psi (approximately 77.3 kgf/cm²). With the above conditions, the value P represented by the equation 1 was 84.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 10 grown specimens (see Fig. 2). The mutation introduction efficiency for the 64 shoot apex specimens was 15.6%.

### (Example 9) Targeted mutagenesis 10 by introduction of gRNA and Cas9 (hyperosmotic treatment 7)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, and the pressure (gas pressure) at the time of shooting was set to 1,350 psi (approximately 94.9 kgf/cm²). With the above conditions, the value P represented by the equation 1 was 103.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 14 grown specimens (see Fig. 2). The mutation introduction efficiency for the 64 shoot apex specimens was 21.9%.

### (Example 10) Targeted mutagenesis 11 by introduction of gRNA and Cas9 (hyperosmotic treatment 8)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used. With the above conditions, the value P represented by the equation 1 was 118.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 13 grown specimens (see Fig. 2). The mutation introduction efficiency for the 64 shoot apex specimens was 20.3%.

### (Example 11) Targeted mutagenesis 12 by introduction of gRNA and Cas9 (hyperosmotic treatment 9)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, and the pressure (gas pressure) at the time of shooting was set to 1,800 psi (approximately 126.5 kgf/cm²). With the above conditions, the value P represented by the equation 1 was 137.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 12 grown specimens (see Fig. 2). The mutation introduction efficiency for the 64 shoot apex specimens was 18.8%.

### (Example 12) Targeted mutagenesis 13 by introduction of gRNA and Cas9 (hyperosmotic treatment 10)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, the pressure (gas pressure) at the time of shooting was set to 1,800 psi (approximately 126.5 kgf/cm²), and the distance from the stopping plate to the target tissue (shoot apices) was set to 33 mm. With the above conditions, the value P represented by the equation 1 was 164.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 7 grown specimens (see Fig. 2). The mutation introduction efficiency for the 58 shoot apex specimens was 12.1%.

### (Example 13) Targeted mutagenesis 14 by introduction of gRNA and Cas9 (hyperosmotic treatment 11)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, the pressure (gas pressure) at the time of shooting was set to 1,800 psi (approximately 126.5 kgf/cm²), and the distance from the stopping plate to the target tissue (shoot apices) was set to 27 mm. With the above conditions, the value P represented by the equation 1 was 299.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 2 grown specimens (see Fig. 2). The mutation introduction efficiency for the 37 shoot apex specimens was 5.4%.

### (Example 14) Targeted mutagenesis 15 by introduction of gRNA and Cas9 (hyperosmotic treatment 12)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, the pressure (gas pressure) at the time of shooting was set to 1,800 psi (approximately 126.5 kgf/cm²), and the distance from the stopping plate to the target tissue (shoot apices) was set to 20 mm. With the above conditions, the value P represented by the equation 1 was 737.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 2 grown specimens (see Fig. 2). The mutation introduction efficiency for the 28 shoot apex specimens was 7.1%.

### (Example 15) Targeted mutagenesis 16 by introduction of gRNA and Cas9 (hyperosmotic treatment 13)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, the pressure (gas pressure) at the time of shooting was set to 1,350 psi (approximately 94.9 kgf/cm²), and the distance from the stopping plate to the target tissue (shoot apices) was set to 10 mm. With the above conditions, the value P represented by the equation 1 was 4,424.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of one grown specimen (see Fig. 2). The mutation introduction efficiency for the 30 shoot apex specimens was 3.3%.

### (Comparative Example 2) Targeted mutagenesis 16 by introduction of gRNA and Cas9 (hyperosmotic treatment 13)

### 1. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of gRNA and Cas9

The gRNA and Cas9 were introduced in the same manner as in (3) of 1 of Example 1, except that gold particles having an average particle size of 1.6 µm were used, the pressure (gas pressure) at the time of shooting was set to 1,800 psi (approximately 126.5 kgf/cm²), and the distance from the stopping plate to the target tissue (shoot apices) was set to 10 mm. With the above conditions, the value P represented by the equation 1 was 5,898.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 0 grown specimens among the 30 shoot apex specimens in the present example, and a genome-edited specimen was not confirmed under the above conditions.

### (Example 16) Gene transfer to target site by introduction of DNA, gRNA, and Cas9

When the cleavage site of the target gene was repaired by introduction of the complex of gRNA and Cas9 into the shoot apex of soybean using the gene transfer method of the present invention, whether a DNA fragment, in which an exogenous gene was inserted, was introduced into a sequence homologous to the target nucleotide sequence of the host was investigated.

### 1. Preparation of donor plasmid used for introduction

### (1) Preparation of exogenous gene

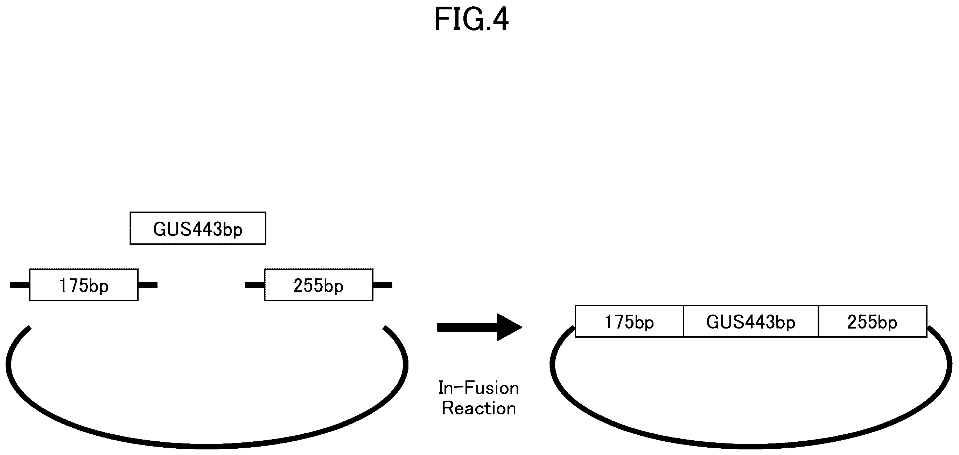

A part (443 bp) of the sequence of GUS gene, which was not present in the plant, was selected as the exogenous gene. The selected sequence was fused with a sequence homologous to the target sequence using In-Fusion (registered trademark) Cloning System (TaKaRa), and the three inserts (exogenous gene and homologous sequences) were cloned into one vector (see Fig. 4).

The exogenous gene was prepared by preparing the following primers specific to a GUS expression vector, and performing PCR.
Primer sequence: ATTTGGAAACGGCAGAGAAG (SEQ ID NO: 5)
Primer sequence: GAGCTCGGTAGCAATTCCC (SEQ ID NO: 6)

As the PCR solution, a solution prepared by adding 1 ng of the GUS expression vector, 25 µL of PrimeSTAR (registered trademark) Max (TaKaRa) Premix (2x), and 0.2 µM each of the primer pair, and filling the resultant mixture up to 50 µL in total with sterile distilled water.

As the PCR, 30 cycles of the reaction were performed using ProFlex PCR System (trademark, ThermoFisher), and each cycle of the reaction included the reaction at 94°C for 1 minute, at 98°C for 10 seconds, at 55°C for 5 seconds, and at 72°C for 15 seconds. After the PCR, 1.5% agarose gel electrophoresis was performed, followed by staining with GelRed, and the DNA of the target size was extracted and purified from the gel using NucleoSpin (registered trademark) Gel and PCR Clean-up (TaKaRa).

### (2) Preparation of homologous sequence

Primers were designed by using the 175 bp upstream from the cleavage site of the soybean target gene as a homologous sequence, and adding a 15 bp homolog (the portions indicated by small letters in the following sequences) to the homologous sequence for fusing with the vector and the exogenous gene.
Primer sequence: gtgctggaattcaggTTGCTGACATCACTCCTCAAGAG (SEQ ID NO: 7)
Primer sequence: ctgccgtttccaaatTGGTACTTTACTTGGGTGATGAC (SEQ ID NO: 8)

For the PCR solution, the homologous sequence was amplified using 100 ng of Yukihomare genome, and was extracted and purified from the gel according to the method described in (1) of 1 of Example 16.

Primers were designed by using the 255 bp downstream from the cleavage site of the soybean target gene as a homologous sequence, and adding a 15 bp homolog (the portions indicated by small letters in the following sequences) to the homologous sequence for fusing with a vector and an exogenous gene.
Primer sequence: attgctaccgagctcAGGGGGCTGTGGTATGTGAAACC (SEQ ID NO: 9)
Primer sequence: tctgcagaattcaggTATCACATTTAACTTTTCAACG (SEQ ID NO: 10)

For the PCR solution, the homologous sequence was amplified using 100 ng of Yukihomare genome, and was extracted and purified from the gel according to the method described in (1) of 1 of Example 16.

### (3) Cloning into vector

In order to clone the above three inserts into a pCR (registered trademark) - Blunt (ThermoFisher) vector, the inserts were mixed with the vector at the same molar concentrations, 5x In-Fusion HD Enzyme Premix was added to the mixture, and the resultant mixture was filled up to 10 µL in total with sterile distilled water. After reacting the resultant solution at 50°C for 15 minutes, the vector was introduced into E. coli by a heat shock method to transform E. coli. In order to select the transformed E. coli, the cells were applied onto an LB agar medium including kanamycin (50 µg/L), and static culture was performed at 37°C for 16 hours.

To determine whether the inserts of interest were inserted into the obtained single colonies, colony PCR was performed using the primers on the vector.
Primer sequence: GTAAAACGACGGCCAG (SEQ ID NO: 11)
Primer sequence: CAGGAAACAGCTATGAC(SEQ ID NO: 12)

As the PCR solution, a solution prepared by adding 20 µL of 2x Quick Taq (registered trademark) HS DyeMix and 0.2 µM each of the primer pair, filling the resultant mixture up to 40 µL in total with sterile distilled water, and picking the single colonies with a sterilized toothpick to suspend was used.

As the PCR, 30 cycles of the reaction were performed using ProFlex PCR System (trademark, ThermoFisher), and each cycle of the reaction included the reaction at 94°C for 2 minutes, at 94°C for 30 seconds, at 55°C for 30 seconds, and at 68°C for 1 minute and 30 seconds. After the PCR, 1.5% agarose gel electrophoresis was performed, followed by staining with GelRed, and the E. coli of the target size was plated on an LB liquid medium, and shale culture was performed at 37°C for 16 hours.

The E. coli was collected from the culture solution, and the plasmid was recovered using QIAprep Spin Miniprep Kit (Qiagen). The recovered plasmid was subjected to a sequence analysis, and the plasmid molecules without PCR errors were cryopreserved as a donor plasmid.

### 2. Preparation of donor DNA used for introduction

On the day of shooting, the donor DNA was amplified with the donor plasmid as a template using high fidelity PCR enzyme, thereby preparing the donor DNA.

As the PCR solution, a solution obtained by adding 1 ng of the above plasmid, 1U of KOD -Plus- Neo, 5 µL of 10x accompanying buffer, 5 µL of 2 mM dNTPs, 3 µL of 25 mM MgSO₄, and 0.2 µM each of the primer pair, and filling the resultant mixture up to 50 µL in total with sterile distilled water was used,

As the PCR, 32 cycles of the reaction were performed using ProFlex PCR System (trademark, ThermoFisher), and each cycle of the reaction included the reaction at 94°C for 2 minutes, at 98°C for 10 seconds, at 55°C for 30 seconds, and at 68°C for 30 seconds. After the PCR, purification was performed using NucleoSpin (registered trademark) Gel and PCR Clean-up (TaKaRa), thereby preparing a donor DNA solution.

### 3. Confirmation of introduction of target gene mutation in T₀ generation plant

### (1) Preparation of mature seeds of soybean

Mature seeds were prepared in the same manner as in (1) of 1 of Comparative Example 1.

### (2) Exposure of shoot apex of mature seed embryo

The shoot apices were exposed in the same manner as in (2) of 1 of Example 3.

### (3) Introduction of DNA, gRNA, and Cas9

Coated fine particles were prepared in the same manner as in (3) of 1 of Comparative Example 1, except that, when Nuclease-Free Water was added, 10 µg of the DNA donor solution was also added to ultimately prepare 26 µL of a solution of the gold particle/Cas9 composite and the DNA donor.

The shooting by the particle gun was performed in the same manner as in (3) of 1 of Example 5, except that the pressure at the time of shooting was set to 650 psi (approximately 45.7 kgf/cm²) or 900 psi (approximately 63.3 kgf/cm²). With the above conditions, the values P represented by the equation 1 were 50 and 69, respectively.

### 4. Investigation using genomic PCR of T₀ generation plant as indicator

### (1) Growth of DNA, gRNA, and Cas9-introduced specimens

The DNA, gRNA, and Cas9-introduced specimens were grown in the same manner as in (1) of 2 of Comparative Example 1.

### (2) Confirmation of introduction of gene into target site in T₀ lower leaf

The presence or absence of the exogenous gene introduced into the target site in the obtained plant was investigated according to the method described in (2) of 2 of Comparative Example 1. After the PCR, 1.5% agarose gel electrophoresis was performed, followed by staining with GelRed. As a result, the PCR product of the wild-type strain was 505 bp, whereas the DNA having a size close to the size (948 bp), which was increased by 443 bp by introduction of the exogenous gene, was detected (see Table 1).

**[Table 1]**

| Shooting pressure (psi) | Number of hypocotyls | Number of grown specimens | Number of gene-introduced specimens |
|---|---|---|---|
| 650 | 640 | 588 | 14 |
| 900 | 192 | 179 | 1 |

The above DNA was extracted from the agarose gel and purified, and were subjected to a sequence analysis to confirm whether the exogenous gene was introduced into the target gene sequence. In addition to the specimen in which only 443 bp of the exogenous gene was inserted, the specimen in which the donor was introduced by the non-homologous end joining was confirmed.

### (3) Confirmation of introduction of gene into target site in T₀ upper leaf

The obtained specimens were transplanted from the cell tray to a pot having a diameter of approximately 10.5 cm, and were grown in a glass greenhouse until compound leaves developed from the third true leaves onward. The confirmation of the presence or absence of the introduced exogenous gene in the upper compound leaves was performed by separately extracting genomic DNA from three independent compound leaves, and confirming the presence or absence of the exogenous gene according to the method described in (2) of 2 of Comparative Example 1.

As a result, the DNA close to the size (943 bp), which was increased by 443 bp by introduction of the gene, was also detected in the upper compound leaf (see Table 2). In the case of the shooting pressure of 900 psi, the introduction of the exogenous gene was detected in the third true leaves and following leaves in the specimen in which the introduction of the exogenous gene was not detected in the second and lower leaves. The DNA was extracted from the agarose gel of the bands of these two specimens and purified, and was subjected to sequence analysis. As a result, only 443 bp of the exogenous gene of interest was inserted. The specimen with which the target mutation was detected in the upper compound leaf was grown, and whether the mutation was inherited to a T₁ plant, which was the next generation, was confirmed.

**[Table 2]**

| Shooting pressure (psi) | Number of hypocotyls | Number of grown specimens | Number of gene-introduced specimens | |
|---|---|---|---|---|
| | | | Lower leaves | Upper leaves |
| 650 | 640 | 588 | 14 | 4 |
| 900 | 192 | 179 | 1 | 2 |

### 5. Confirmation of introduction of gene into target site in T₁ generation plant

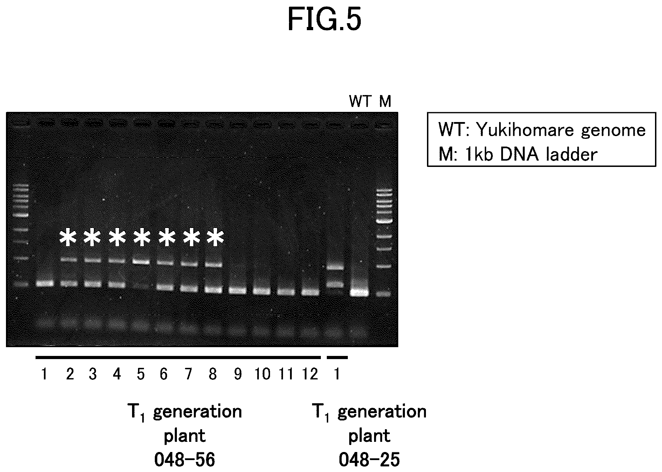

T₁ seeds harvested from the T₀ plant were sown, primary leaves were sampled, and genomic DNA was extracted from the primary leaves by the benzyl chloride method. Then, the confirmation of introduction of the exogenous gene into the target site was performed according to the method described in (2) of 2 of Comparative Example 1. As a result, the introduction of the target gene mutation was confirmed in the T₁ generation primary leaf of one line of the T₁ generation of the shooting pressure of 650 psi. The DNA was extracted from the agarose gel of the band of this one line and purified, and was subjected to sequence analysis. As a result, only 443 bp of the exogenous gene of interest was clearly inserted (see Fig. 5).

In Fig. 5, the samples indicated with asterisks are the T₁ plants in which the target gene is introduced into the target site by genome editing.

The gene introduction efficiency (the number of specimens in which the gene was introduced/the number of shoot apices × 100) per shoot apex on which the gene introduction treatment was performed was calculated from the obtained specimens in which the gene was introduced, and the result was 0.16%.

It was found from the above that genome editing was performed and the genome-edited plant was produced with high efficiency by setting the value P represented by the equation 1 to the predetermined range when the fine particles coated with the nucleic acid and the protein were shot into the shoot apex of the plant using the gene gun equipped with the stopping plate.

### (Example 17) Introduction of DNA, gRNA, and Cas9 into shoot apex tissue of maize

### 1. Introduction of DNA, gRNA, and Cas9 into maize shoot apex tissue

### (1) Preparation of maize mature seeds

Mature seeds of maize (variety: A188) were immersed in 70%(w/w) ethanol for 1 minutes, and then were agitated in a 2%(w/v) aqueous sodium hypochlorite solution for 10 minutes, thereby sterilizing the mature seeds. Then, the seeds were washed with sterile water three times, and the washed seeds were soaked in sterile water. The resultant seeds were incubated at 30°C for 24 hours, and then were provided to the following experiment.

### (2) Exposure of shoot apex of mature seed embryo

Under a stereoscopic microscope, the endosperm and excess scutellum portions in the mature seeds were removed by a sterilized knife (a sterilized scalpel). Then, coleoptiles and leaf primordia of the embryo portions of the germinated seeds were removed using a tip of a needle (diameter: 0.20 mm) to fully expose the shoot apex portions. The shoot apex portions were placed on a permeable bombardment plate (MSOSM medium: MS inorganic salt, MS vitamin, 20 g/L sucrose, 36.4 g/L mannitol, 36.4 g/L sorbitol, 0.93 g/L MES, 3% PPM (plant preservative mixture, registered trademark, NACALAI TESQUE, INC.), 7.0 g/L phytagel, pH 5.8), and were left to stand in the dark at 25°C for 24 hours.

### (3) Introduction of DNA, gRNA, and Cas9

Introduction of gRNA and Cas9 into maize shoot apices was performed by particle bombardment in the following manner.

As fine particles, 30 mg of gold particles having an average particle size of 1.0 µm were weighed and added to 500 µL of 70% ethanol, and the resultant mixture was sufficiently suspended by vortexing. Thereafter, the gold particles were precipitated by centrifugation, and the ethanol was removed. Thereafter, 250 µL of sterile water was added to prepare a sterilized gold particle solution.

In the present example, Alt-R CRISPR-Cas9 sgRNA including a recognition sequence (SEQ ID NO: 13) (Integrated DNA Technologies) (1.0 µg/µL) was used as gRNA. As the Cas9, Cas9 derived from Streptococcus pyogenes (Takara Bio Inc.) was used.
Recognition sequence: UGAGCCAGAUGCGCUCCUCG (SEQ ID NO: 13)

To 2,400 µg of gold particles, a mixed solution including 5 µL of the gRNA, 12.8 µg of the Cas9, 3 µg of plasmid DNA including a fluorescent reporter gene sGFP (S65T) sequence expressed under the control of a maize ubiquitin promoter and a first intron (pZmUbi-sGFPS65T), 4 µL of 10× Cut Smart (registered trademark) Buffer (New England Biolabs), 1.0 µL of Ribolock RNase Inhibitor (ThermoFisher), and 5 µL of a 1,4-bis(3-oleoylamidopropyl)piperazine/histone H1 protein mixture (3:1, 1,330 µg/mL). After discarding the supernatant, 24 µL of Nuclease-Free Water was added, and the resultant was provided as a gold particle/Cas9 composite. An amount of the gold particles per shot was set to 600 µg. In a clean bench, a hydrophilic film (3M, SH2CLHF) cut into a square with a side of 1.5 cm was adhered to a center of a macrocarrier. To each hydrophilic film, 6 µL of the gold particle/Cas9 composite was poured, and was air-dried.

As the particle gun, Biolistic (registered trademark) PDS-1000/He Particle Delivery System (Bio-RAD) was used.

The pressure (gas pressure) at the time of shooting was set to 1,800 psi (approximately 126.5 kgf/cm²), and the distance from the stopping plate to the target tissue (shoot apices) was set to 35 mm. With the above conditions, the value P represented by the equation 1 was 13.12.

The fine particles were shot four times per dish, and then the resultant shoot apex portions were allowed to stand overnight in the dark.

### 2. Investigation of introduction of substance in T₀ generation plant

### (1) Investigation of introduction of substance in treated specimen

GFP fluorescence in the shoot apices was observed under a fluorescence stereomicroscope (stereomicroscope SZX7 manufactured by Olympus Corporation, fluorescence observation lighting U-HGLGPS, GFP mirror unit SZX-MGFPA, CCD camera DP73), and the specimens in which the substance was successfully introduced were observed. Among the specimens (treated specimens) to which the substance introduction treatment was performed by the particle bombardment, the shoot apex tissue in which GFP fluorescence was observed at one or more spots was determined as a substance-transferred specimen. Among 45 hypocotyls subjected to the treatment (may be referred to as "treated specimens" hereinafter), the number of the hypocotyls in which the GFP fluorescence was confirmed in the shoot apex (may be referred to as a "substance-introduced specimen number" hereinafter) was one.

### (2) Growth of substance-introduced specimen

The substance-introduced specimens were transferred onto a germination medium (MS inorganic salt, MS vitamin, 20 g/L sucrose, 1 g/L MES, 3% PPM, 7.0 g/L phytagel, 5 µM AgNO₃, 10 µM CuSO₄, pH 5.8), and were cultured under light at 25°C. After 2 weeks, the specimens in which hypocotyls were regularly growing were determined as viable substance-introduced specimens.

The number of the treated specimens, the number of the substance-introduced specimens, and the number of the viable substance-introduced specimens were counted. The results are presented in Table 3.

### (Comparative Example 3) Introduction of DNA, gRNA, and Cas9 into shoot apex tissue of maize

### 1. Introduction of DNA, gRNA, and Cas9 into maize shoot apex tissue (1) Preparation of maize mature seeds

Mature seeds were prepared in the same manner as in (1) of 1 of Example 17.

### (2) Exposure of shoot apex of mature seed embryo

The shoot apex was exposed in the same manner as in (2) of 1 of Example 17.

### (3) Introduction of DNA, gRNA, and Cas9

The gRNA and Cas9 were introduced into maize shoot apices in the same manner as in (3) of 1 of Example 17, except that gold particles having an average particle size of 0.6 µm were used as the fine particles, the pressure (gas pressure) at the time of shooting was set to 1,550 psi (approximately 108.9 kgf/cm²), and the distance from the stopping plate to the target tissue (shoot apices) was set to 50 mm. With the above conditions, the value P represented by the equation 1 was 0.30.

### 2. Investigation of introduction of substance in T₀ generation plant

### (1) Investigation of introduction of substance in treated specimen

The investigation was performed in the same manner as in (1) of 2 of Example 17. As a result, among the 48 hypocotyls subjected to the treatment, the number of the hypocotyls in which the GFP fluorescence was confirmed in the shoot apex was 19.

### (2) Growth of substance-introduced specimen

The substance-introduced specimens were grown in the same manner as in (2) of 2 of Example 17.

The number of the treated specimens, the number of the substance-introduced specimens, and the number of the viable substance-introduced specimens were counted. The results are presented in Table 3.

**[Table 3]**

| | Ex. 17 | Comp. Ex. 3 |
|---|---|---|
| Average particle size of fine particles (µm) | 1.0 | 0.6 |
| Shooting pressure (psi) | 1,800 | 1,550 |
| Distance from stopping plate to target tissue (shoot apex) (mm) | 35 | 50 |
| Value P (Equation 1) | 13.12 | 0.30 |
| Number of treated specimens | 45 | 48 |
| Number of substance-introduced specimens | 1 | 19 |
| Number of viable substance-introduced specimens | 0 | 12 |

In the case where the substance was introduced into 45 shoot apices of the hypocotyls of maize under the condition that the value P of the equation was 13.12 (Example 17), the number of the specimens in which the substance was successfully introduced was only one, from which the grown specimen was not obtained. Conversely, in the case where the substance was introduced into 48 shoot apices of the hypocotyls of maize under the condition that the value P of the equation was 0.30 (Comparative Example 3), the number of the specimens in which the substance was successfully introduced was 19, from which the number of the grown specimens was 12. It was demonstrated from the above results that, when the substance was introduced into the shoot apex, it was difficult to introduce the substance into the shoot apex cells under the condition that the value P of the equation 1 was 0.8 or greater and the growth of the shoot apex tissue was hindered in case of the monocotyledons including maize, and therefore the above condition was not suited for production of genome-edited cells and genome-edited plants. In addition, for the dicotyledons including Glycine max, the condition that the value P of the equation 1 was 0.8 or greater was suitable for introduction of the substance into the shoot apices, which was the characteristic unique to the dicotyledons and was different from the monocotyledons.

### (Example 18) Targeted mutagenesis by introduction of gRNA and Cas9

### 1. Confirmation of introduction of target gene mutation in T₀ generation plant

### (1) Preparation of mature seeds of soybean

Mature seeds were prepared in the same manner as in (1) of 1 of Comparative Example 1, except that "Osuzu" was used as the variety of the soybean.

### (2) Exposure of shoot apex of mature seed embryo

The shoot apices were exposed in the same manner as in (2) of 1 of Example 3.

The size of the growth point (shoot apical meristem) at the tip of the stem was 324 µm in diameter (the average value of the 12 measured shoot apices).

### (3) Introduction of gRNA and Cas9

The gRNA and Cas9 were introduced in the same manner as in (3) of 1 of Example 1, except that gold particles having the average particle size of 1.0 µm were used, and the pressure (gas pressure) at the time of shooting was changed to 1,800 psi (approximately 126.5 kgf/cm²). With the above conditions, the value P represented by the equation 1 was 13.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Comparative Example 1.

### (2) Confirmation of introduction of target gene mutation in T₀ upper compound leaf

The obtained specimens were transplanted from the cell tray to a a pot having a diameter of approximately 10.5 cm, and were grown in a glass greenhouse until compound leaves developed from the third true leaves onward. The confirmation of the presence or absence of the mutation in the upper compound leaves was performed by separately extracting genomic DNA from three independent compound leaves, and confirming the presence or absence of the target gene mutation according to the method described in (2) of 2 of Comparative Example 1.

As a result, the target gene mutation was detected in several upper compound leaves of 15 grown specimens (see Fig. 2). The mutation introduction efficiency for the 128 shoot apex specimens was 11.7%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant

T₁ seeds harvested from the T₀ plant were sown, primary leaves were sampled, and genomic DNA was extracted from the primary leaves by the benzyl chloride method. Then, the confirmation of introduction of the target gene mutation was performed and evaluated by the CAPS assay according to the method described in (2) of 2 of Comparative Example 1. As a result, the introduction of the target gene mutation was confirmed in the T₁ generation primary leaf of 8 lines of the T₁ generation (mutation introduction efficiency: 6.3%) (see Fig. 3).

### (Example 19) Targeted mutagenesis by introduction of gRNA and Cas9

### 1. Confirmation of introduction of target gene mutation in T₀ generation plant

### (1) Preparation of mature seeds of soybean

Mature seeds were prepared in the same manner as in (1) of 1 of Example 18, except that "Yukihomare" was used as the variety of the soybean.

### (2) Exposure of shoot apex of mature seed embryo

The shoot apices were exposed in the same manner as in (2) of 1 of Example 18.

The size of the growth point (shoot apical meristem) at the tip of the stem was 317 µm in diameter (the average value of the 10 measured shoot apices).

### (3) Introduction of gRNA and Cas9

The gRNA and Cas9 were introduced in the same manner as in (3) of 1 of Example 18.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Example 18.

### (2) Confirmation of introduction of target gene mutation in T₀ upper compound leaf

The confirmation of the introduction of the target gene mutation was performed in the same manner as in (1) of 2 of Example 18.

As a result, the target gene mutation was detected in several upper compound leaves of 19 grown specimens (see Fig. 2). The mutation introduction efficiency for the 96 shoot apex specimens was 19.8%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant leaf

The confirmation of introduction of the target mutation was performed in the same manner as in 3 of Example 18.

As a result, the introduction of the target gene mutation was confirmed in the T₁ generation primary leaf of 14 lines of the T₁ generation (mutation introduction efficiency: 14.6%) (see Fig. 3).

### (Example 20) Targeted mutagenesis by introduction of gRNA and Cas9

### 1. Confirmation of introduction of target gene mutation in T₀ generation plant

### (1) Preparation of mature seeds of soybean

Mature seeds were prepared in the same manner as in (1) of 1 of Example 18, except that "Suzumaru" was used as the variety of the soybean.

### (2) Exposure of shoot apex of mature seed embryo

The shoot apices were exposed in the same manner as in (2) of 1 of Example 18.

The size of the growth point (shoot apical meristem) at the tip of the stem was 279 µm in diameter (the average value of the 12 measured shoot apices).

### (3) Introduction of gRNA and Cas9

The gRNA and Cas9 were introduced in the same manner as in (3) of 1 of Example 18.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Example 18.

### (2) Confirmation of introduction of target gene mutation in T₀ upper compound leaf

The confirmation of the introduction of the target gene mutation was performed in the same manner as in (1) of 2 of Example 18.

As a result, the target gene mutation was detected in several upper compound leaves of 11 grown specimens (see Fig. 2). The mutation introduction efficiency for the 90 shoot apex specimens was 12.2%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant leaf

The confirmation of introduction of the target mutation was performed in the same manner as in 3 of Example 18.

As a result, the introduction of the target gene mutation was confirmed in the T₁ generation primary leaf of 6 lines of the T₁ generation (mutation introduction efficiency: 6.7%) (see Fig. 3).

### (Example 20) Targeted mutagenesis by introduction of gRNA and Cas9

### 1. Confirmation of introduction of target gene mutation in T₀ generation plant

### (1) Preparation of mature seeds of soybean

Mature seeds were prepared in the same manner as in (1) of 1 of Example 18, except that "Natto small grains" was used as the variety of the soybean.

### (2) Exposure of shoot apex of mature seed embryo

The shoot apices were exposed in the same manner as in (2) of 1 of Example 18.

The size of the growth point (shoot apical meristem) at the tip of the stem was 212 µm in diameter (the average value of the 12 measured shoot apices).

### (3) Introduction of gRNA and Cas9

The gRNA and Cas9 were introduced in the same manner as in (3) of 1 of Example 18.

### 2. Investigation using genomic PCR of T₀ generation plant as indicator

### (1) Growth of gRNA and Cas9-introduced specimens

The gRNA and Cas9-introduced specimens were grown in the same manner as in (1) of 2 of Example 18.

### (2) Confirmation of introduction of target gene mutation in T₀ upper compound leaf

The confirmation of the introduction of the target gene mutation was performed in the same manner as in (1) of 2 of Example 18.

As a result, the target gene mutation was detected in several upper compound leaves of 11 grown specimens (see Fig. 2). The mutation introduction efficiency for the 144 shoot apex specimens was 7.6%. The specimen in which the target mutation was detected in the upper compound leaf was grown, and whether the mutation was inherited to a T₁ plant, which was the next generation, was confirmed.

### 3. Confirmation of introduction of target gene mutation in leaf of T₁ plant leaf

The confirmation of introduction of the target mutation was performed in the same manner as in 3 of Example 18.

As a result, the introduction of the target gene mutation was confirmed in the T₁ generation primary leaf of 5 lines of the T₁ generation (mutation introduction efficiency: 3.5%) (see Fig. 3).

As demonstrated in Examples 18 to 21, it was found that, even when the sizes of the growth points (shoot apical meristem) at the tips of the stems of the plants were different, genome editing was performed or the genome-edited plant was produced with high efficiency, if the value P of the equation 1 was satisfied.

For example, embodiments of the present invention include the following.
<1> A genome editing method including:
   a step of shooting fine particles coated with at least one nucleic acid and at least one protein into a shoot apex of a plant using a gene gun equipped with a stopping plate,
   wherein a value P represented by an equation 1 below is 0.8 or greater and 5,000 or less: $P = {\left(A/2\right)}^{5} \times B \times {10}^{4} / {C}^{3}$
   where, in the equation 1, A is "an average particle size (µm) of the fine particles", B is "a gas pressure (psi) of the gene gun", and C is "a distance (mm) between the stopping plate of the gene gun and the shoot apex".
<2> The genome editing method according to <1>, further including: a step of bringing the shoot apex into contact with a hyperosmotic medium.
<3> The genome editing method according to <1> or <2>,
   wherein the plant is a dicotyledon.
<4> The genome editing method according to <3>,
   wherein the dicotyledon is a plant of the Fabaceae.
<5> The genome editing method according to any one of <1> to <4>, further including:
   a step of growing the shoot apex shot with the fine particles.
<6> The genome editing method according to any one of <1> to <5>,
   wherein the at least one nucleic acid includes a gene of interest to be introduced into a genome of the plant.
<7> The genome editing method according to any one of <1> to <6>,
   wherein the value P represented by the equation 1 is 0.8 or greater and 250 or less.
<8> A method for producing a genome-edited plant, including:
   a step of shooting fine particles coated with at least one nucleic acid and at least one protein into a shoot apex of a plant using a gene gun equipped with a stopping plate; and
   a step of growing the shoot apex shot with the fine particles,
   wherein a value P represented by an equation 1 below is 0.8 or greater and 5,000 or less: $P = {\left(A/2\right)}^{5} \times B \times {10}^{4} / {C}^{3}$
   where, in the equation 1, A is "an average particle size (µm) of the fine particles", B is "a gas pressure (psi) of the gene gun", and C is "a distance (mm) between the stopping plate of the gene gun and the shoot apex".
<9> The method for producing the genome-edited plant according to <8>, further including:
   a step of bringing the shoot apex into contact with a hyperosmotic medium.
<10> The method for producing the genome-edited plant according to <8> or <9>,
   wherein the plant is a dicotyledon.
<11> The method for producing the genome-edited plant according to <10>,
   wherein the dicotyledon is a plant of the Fabaceae.
<12> The method for producing the genome-edited plant according to any one of <8> to <11>,
   wherein the at least one nucleic acid includes a gene of interest to be introduced into a genome of the plant.
<13> The method for producing the genome-edited plant according to any one of <8> to <12>,
   wherein the value P represented by the equation 1 is 0.8 or greater and 250 or less.

This international application claims priority based on Japanese Patent Application No. 2023-177413 filed on October 13, 2023, the entire contents of which are incorporated herein by reference.

## Claims

1. A genome editing method comprising:
a step of shooting fine particles coated with at least one nucleic acid and at least one protein into a shoot apex of a plant using a gene gun equipped with a stopping plate,
wherein a value P represented by an equation 1 below is 0.8 or greater and 5,000 or less: $P = {\left(A/2\right)}^{5} \times B \times {10}^{4} / {C}^{3}$
where, in the equation 1, A is "an average particle size (µm) of the fine particles", B is "a gas pressure (psi) of the gene gun", and C is "a distance (mm) between the stopping plate of the gene gun and the shoot apex".

2. The genome editing method according to claim 1, further comprising:
a step of bringing the shoot apex into contact with a hyperosmotic medium.

3. The genome editing method according to claim 1,
wherein the plant is a dicotyledon.

4. The genome editing method according to claim 3,
wherein the dicotyledon is a plant of the Fabaceae.

5. The genome editing method according to claim 1, further comprising:
a step of growing the shoot apex shot with the fine particles.

6. The genome editing method according to claim 1,
wherein the at least one nucleic acid includes a gene of interest to be introduced into a genome of the plant.

7. The genome editing method according to claim 1,
wherein the value P represented by the equation 1 is 0.8 or greater and 250 or less.

8. A method for producing a genome-edited plant, comprising:
a step of shooting fine particles coated with at least one nucleic acid and at least one protein into a shoot apex of a plant using a gene gun equipped with a stopping plate; and
a step of growing the shoot apex shot with the fine particles,
wherein a value P represented by an equation 1 below is 0.8 or greater and 5,000 or less: $P = {\left(A/2\right)}^{5} \times B \times {10}^{4} / {C}^{3}$
where, in the equation 1, A is "an average particle size (µm) of the fine particles", B is "a gas pressure (psi) of the gene gun", and C is "a distance (mm) between the stopping plate of the gene gun and the shoot apex".

9. The method for producing the genome-edited plant according to claim 8, further comprising:
a step of bringing the shoot apex into contact with a hyperosmotic medium.

10. The method for producing the genome-edited plant according to claim 8,
wherein the plant is a dicotyledon.

11. The method for producing the genome-edited plant according to claim 10,
wherein the dicotyledon is a plant of the Fabaceae.

12. The method for producing the genome-edited plant according to claim 8,
wherein the at least one nucleic acid includes a gene of interest to be introduced into a genome of the plant.

13. The method for producing the genome-edited plant according to claim 8,
wherein the value P represented by the equation 1 is 0.8 or greater and 250 or less.
